# EUROPEAN PATENT APPLICATION

(11) **EP 4 810 496 A1**
(43) Date of publication of application: **23.09.2026**
(21) Application number: 25165357.2
(22) Date of filing: 21.03.2025
(51) Int. Cl.: C07D 473/18, A61P 29/00, A61P 31/04, A61P 31/22, A61P 35/00, C07D 487/04, A61K 31/52, A61K 31/53

(54) **SUBSTITUTED IMIDAZO[2,1-F][1,2,4]-TRIAZINES AS CDK7 INHIBITORS**

(71) Applicant: Universität Münster, 48149 Münster (DE)
(72) Inventor: MEISTERERNST, Michael, 48149 Münster (DE)
(74) Representative: Kutzenberger Wolff & Partner

(57) **Abstract**

The invention relates to substituted Imidazo[2,1-f][1,2,4]-triazines as CDK7 Inhibitors.

## Description

The invention relates to substituted Imidazo[2,1-f][1,2,4]-triazines as CDK7 inhibitors and medicaments comprising the substituted Imidazo[2,1-f][1,2,4]-triazines. Further, the invention relates to substituted Imidazo[2,1-f][1,2,4]-triazines for use in the treatment of proliferative diseases, preferably cancers.

Cyclin-dependent kinase (CDK) family members that trigger passage through the cell cycle are being considered as attractive therapeutic targets, especially for cancer. CDK family members that control other processes such as transcription and RNA processing have caught less attention so far, although experimental evidence for their involvement in different pathological processes is emerging. Together with cell cycle control, CDK/cyclin complexes also have been identified as conserved components of the RNA polymerase II (Pol II) transcriptional machinery. There are currently 20 known mammalian CDKs. While CDK7 - 13 have been linked to transcription, only CDK 1, 2, 4, and 6 show demonstrable association with cell cycle. Unique among the mammalian CDKs, CDK7 has consolidated kinase activities, regulating both the cell cycle progression and transcription.

The general transcription factor TFIIH purified from mammalian cells consists of ten subunits, seven of which (p62, p52, p44, p34, XPD, XPB, and TTDA) form the core complex. Three subunits (cyclin H, MAT1, and CDK7) form the CDK7 complex which is linked to TFIIH's core via the XPD (ATP-dependent helicase) subunit of complex. During the process of transcription initiation, the helicase activity of TFIIH opens the core promoter DNA, while CDK7 phosphorylates the C-terminal domain (CTD) of Pol II at serine 5 and 7 as well as other transcription factors controlling the initiation-to-elongation transition and RNA stability. Therefore, CDK7 is an essential factor for transcription process, which suggests that CDK7 is a target for cancer therapy, especially transcription addicted cancer.

By mediating T loop phosphorylation CDK7 is further thought to positively control the activity of the entire CDK family. It is, hence, considered to be a CDK-activating kinase (CAK) that will influence the specific impact of individual members of the CDK family. This relates for example to CDK9 inhibition (mediated i.e. through flavopiridol and more specific CDK9 inhibitors like Enitociclib) that leads to down-regulation of a large number of short-lived anti-apoptotic proteins, such as the anti-apoptotic proteins myeloid cell leukemia-1 (Mcl-1), B-cell lymphoma extra-long (Bcl-xL) and XIAP (X-linked IAP), D-cyclins, c-Myc, Mdm-2 (leading to p53 stabilization), p21^{wafl} proteins whose transcription is mediated by nuclear factor-kappa B (NF-kB) and hypoxia-induced VEGF. These findings supported previous postulates that CDK7 might be a valuable target for drugs directed toward the treatment of malignancies and cell cycle-associated diseases.

The function of CDK7 as regulator of general transcription and as a therapeutic target for treatment of many diseases and syndromes is associated with mutations in regulatory regions and in transcription factors, cofactors, chromatin regulators and noncoding RNAs. These mutations can contribute to cancer, autoimmunity, neurological disorders, developmental syndromes, diabetes, cardiovascular disease, and obesity, among others. Some transcription factors control RNA polymerase II pause release and elongation and, when their expression or function is altered, can produce aggressive tumor cells (c-Myc) or some forms of autoimmunity (AIRE). Therefore, inhibition of human CDK7 kinase activity is likely to result in anti-proliferative activity through the function in cell cycle progression and transcriptional regulation by inhibition of some transcription factor related to oncogene through inhibition of general transcription process. Importantly, CDK7 was also shown to regulate expression of oncogenic transcription factors more dramatically than other housekeeping genes in cancer cells. Thus, inhibition of CDK7 can differentially affect transcription of certain cell cycle and oncogenes, therefore a therapeutic window can be secured. For this reason, transcriptional regulation and pharmacological inhibition through appropriate general transcription inhibition by CDK7 could be applied to treat proliferative disorder, including cancer. As a general regulator of transcription, CDK7 is a therapeutic target for treatment of disease like inflammation, virus replication such as HIV, CMV and EBV, cancer and cardiac hypertrophy.

HIV-l gene expression is regulated by the viral transactivator protein (Tat) which induces transcriptional elongation of HIV-l long tandem repeat. This induction requires hyperphosphorylation of the C-terminal domain repeat of RNA polymerase II. To archives said hyperphosphorylation, Tat stimulates CTD kinases associated with general transcription factors of the promoter complex, specifically TFIIH-associated CDK7. It has been described that Tat binds to CDK7 and that this interaction increases the ability of CAK to phosphorylate CTD. It has further been described that the transcriptional activation by Tat is dependent upon the kinase activity of CDK7. Additionally, it has been concluded that the recruitment and activation of TFIIH represents a rate-limiting step for the emergence of HIV from latency.

Levels of CDK7 and CDK9, as well as other components of the kinase complexes, MAT-1/cyclin H are upregulated during Human cytomegalovirus infection. In addition, there is an increase in the kinase activities of CDK7 and CDK9.

Many antiviral drugs target viral proteins. These have the disadvantage that viruses often develop resistance against these drugs. Antiviral drugs targeting cellular proteins essential for viral process, like CDK7, could bypass this disadvantage. These drugs may further be effective in treating several unrelated viruses and their effects should be additive to traditional antiviral agents. Inhibitors of CDK7, which has its dual function of CDK-activating kinase and transcription regulation is very effective in the treatment of several viruses.

WO 2019/197549 A1 discloses pyrazolo[1,5-a][1,3,5]triazine and pyrazolo[1,5-a]pyrimidine derivatives and/or pharmaceutically acceptable salts thereof, the use of these derivatives as pharmaceutically active agents, especially for the prophylaxis and/or treatment of cell proliferative diseases, inflammatory diseases, immunological diseases, cardiovascular diseases and infectious diseases. Furthermore, WO 2019/197549 A1 discloses pharmaceutical compositions containing at least one of the pyrazolo[1,5-a][1,3,5]triazine and pyrazolo[1,5-a]pyrimidine derivatives and/or pharmaceutically acceptable salts thereof.

WO 2020/186196 A1 discloses compounds having activity as cancer agents.

WO 2022/117504 A1 discloses further compounds having activity as cancer agents.

EP 4 269 403 A1 discloses further compounds having activity as cancer agents.

The CDK7 inhibitors according to the prior art exhibit disadvantages. E.g., the CDK7 inhibitors according to the prior art exhibit limited specificity and affinity. Further, viruses often develop resistance against the CDK7 inhibitors according to the prior art.

It is an object of the invention to provide compounds that have advantages compared to the compounds of the prior art.

This object has been achieved by the subject-matter of the patent claims.

It has been surprisingly found that the CDK7 inhibitors according to the invention exhibit excellent selectivity and/or affinity for CDK7.

In particular, it has been surprisingly found that the side chains of the compounds of the present invention contribute to improved selectivity and/or affinity for CDK7: It has been surprisingly found that an -O-R group at the position of R3 according to the compounds of the present invention contributes to improved affinity, while a piperazine group at the position of **R1** according to the compounds of the present invention contributes to improved selectivity. Furthermore, it has been surprisingly found that an -CHF₂ group at the position of R7, R8 and R9 according to the compounds of the present invention works in cell walls and may be beneficial in organisms.

It has been surprisingly found that compound TBM-C013 selectivity for CDK7 over the closely related CDK2.

It has been surprisingly found that derivates of TBM-C013 represented in TBM-C021 and TBM-C019 _1 lead to even higher selectivity of CDK7 over the closely related CDK2.

It has been surprisingly found that the piperidine-substituted compounds (see position **R1** according to the compounds of the present invention) exhibit high affinity for CDK7. Modified pyrazine-triazines (specifically compound TBM-C054) achieve a comparable affinity in the low nanomolar range, however, with much higher specificity for CDK7. Compound TBM-C054 binds about three log levels better to CDK7 than to the next related kinase CDK2, while the piperidine-adenine compound TBM-C002 and also the piperidine-triazine compound LDC4297 bind slightly more than one order of magnitude better to CDK7 than to CDK2.

It has been surprisingly found that especially compounds TBM-C054 and TBM-C050 are efficient in the treatment of pancreatic cancer.

It has been surprisingly found that compound TBM-C055 opens up a chemical way to dock the inhibitors to other molecules and cells. The primary intention is to improve stability through a PEG module in the organism. In principle, after further chemical modification (e.g. attachment of mevalonate or other "click modules"), the substance enables targeted docking to tumor cells through covalent binding by binding to antibodies or specific nanoparticles.

In addition, based on reported cell cycle phenotypes, tumors with upregulated cell cycle genes such as CyclinD1 or even CDK7 itself could be preferred targets. Related, but specifically of interest, is also the massive downregulation of the Myc oncogene by CDK7 inhibitors. This observation qualifies tumors with overexpressed Myc as specific targets.

Among viruses, especially DNA viruses and those that depend on cellular RNA polymerase II activity for efficient replication are potential targets. Herpes viruses such as EBV and CMV are relevant targets. These viruses remain latent in the organism and become a problem in immunocompromised patients.

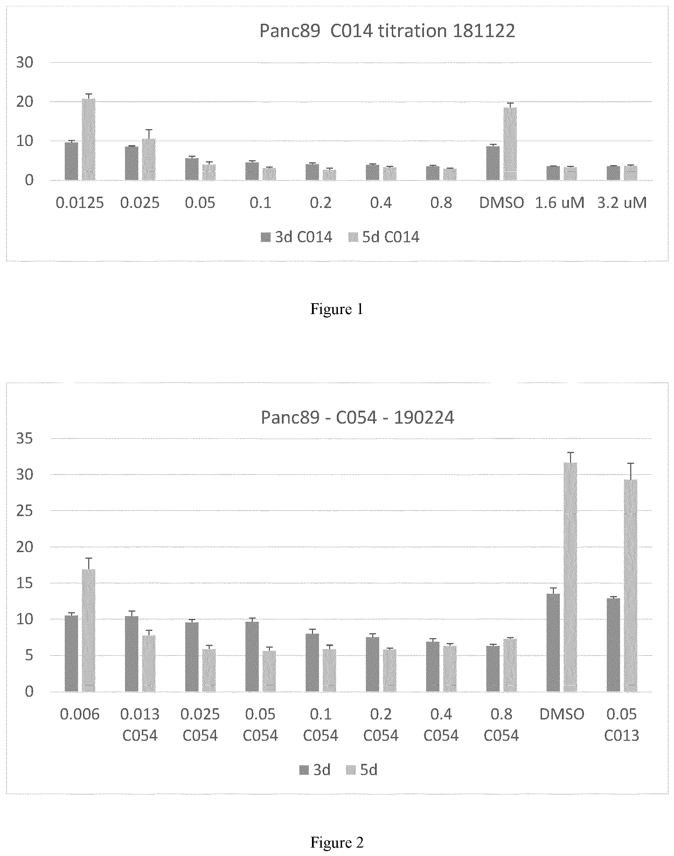

Figure 1 shows the relative growth level of the pancreatic cancer cell line (Panc89) (Y-axis) in response to increasing concentrations (X-axis) of the adenine derivative TBM-C014 (compare with DMSO control). Proliferation levels are indicated as multiples of starting levels at day 0. 3d, 5d indicate 3 days and 5 days incubation periods at 37°C, respectively. Concentrations are always given in uM. Measurements of growth rates were generally conducted with CellTiterGlo ^{®} assay (Promega).
Figure 2 displaying superior inhibition of Panc89 growth by the 1,2,4 triazine TBM-C054 (ID50_5d about 6 nM), a compound which is identical in the side chains to TBM-C014 but differs in the core base.

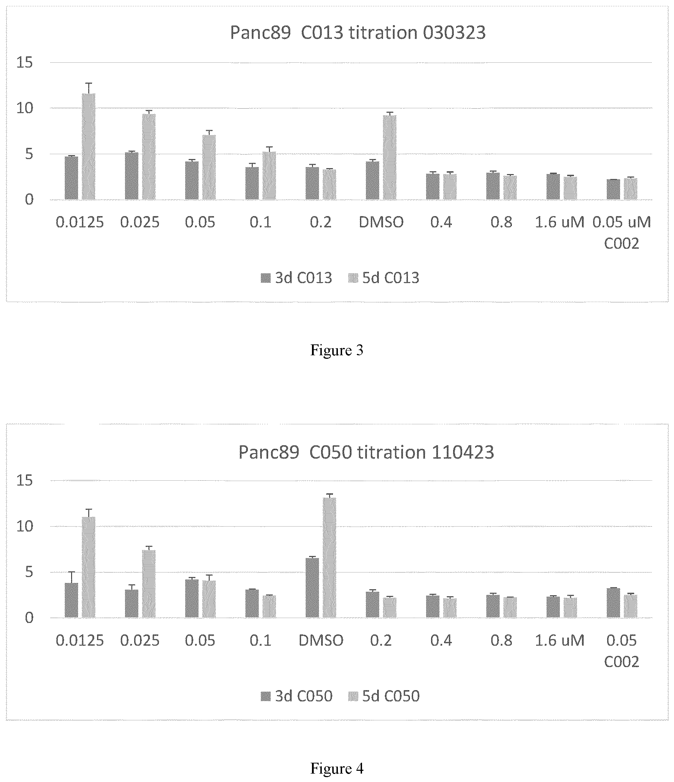

Figure 3 shows an analogues experiment demonstrating inhibition by the adenine-piperazine derivative TBM-C013 lacking the O-Me side chain present in C014.
Figure 4 demonstrates more efficient inhibition of Panc89 by the 1,2,4 triazine-piperazine derivative TBM-C050, compared with the corresponding adenine-derivative TBM-C013.

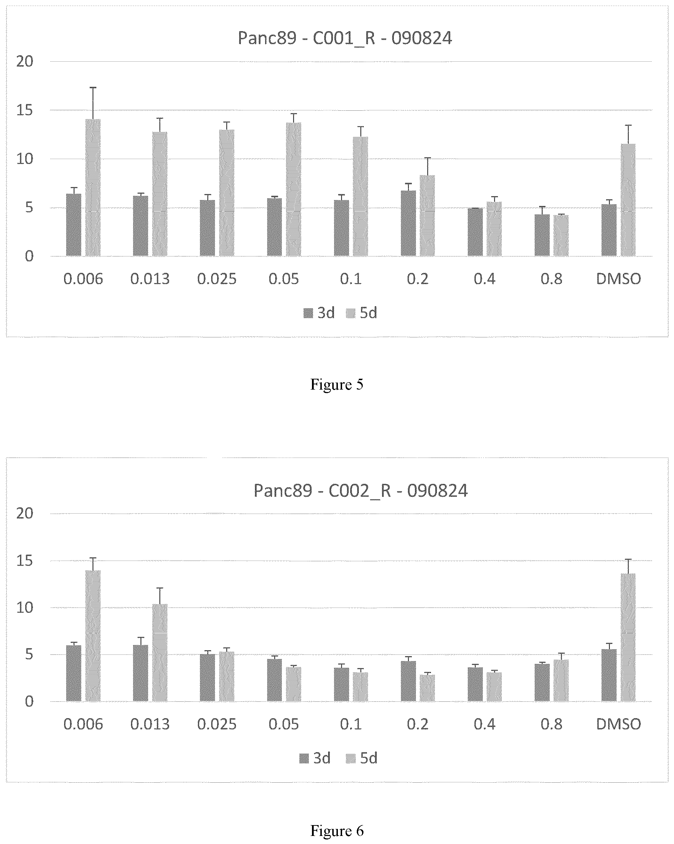

Figure 5 shows inhibition of cell line Panc89 by compound C001_R a pyrazolo[1,5-a] pyrimidine with corresponding side chains to C002-R.
Figure 6 Superior inhibition of Panc89 by C002-R the corresponding adenine derivative (pyrazolo[1,5-a] pyrimidine derivative C001_R (Fig. 5) elicits significantly lower affinity than the corresponding adenine derivative C002_R (Fig.6)).

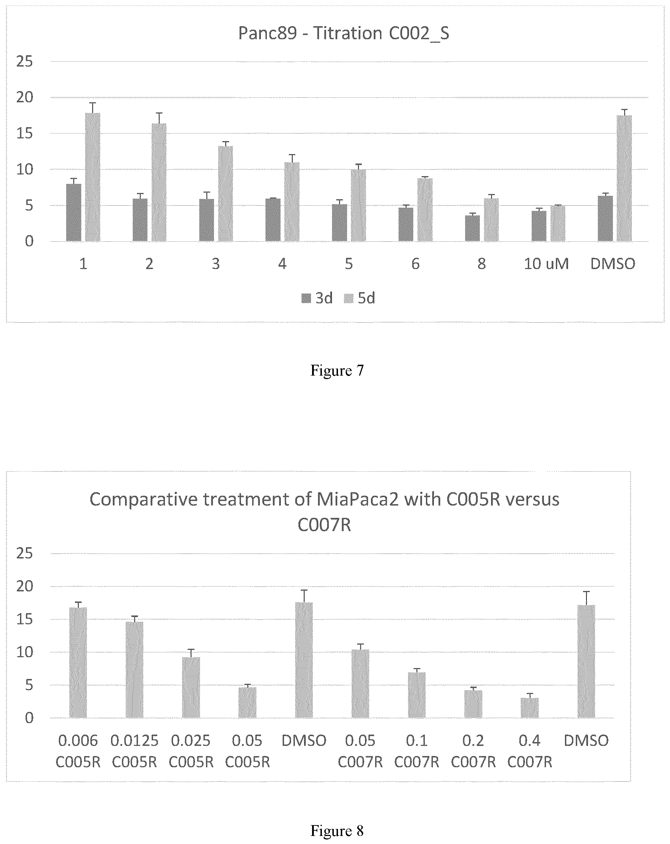

Figure 7 Titration of S variant of C002 on Panc89 cells reveals an approximately 100-fold reduced affinity as compared with the R-form of C002 (C002-R (Fig. 6) dramatically outperforms C002-S (Fig.7))
Figure 8 C005R versus C007R: Approximately 4-fold enhanced impact of Pyrazol side chain compared with -N(Me)₂ side chain (-Iospropyl Pyrazol side chain in TBM-C005R outperforms corresponding -N(CH₃)₂ side chain in TBM-C007R)

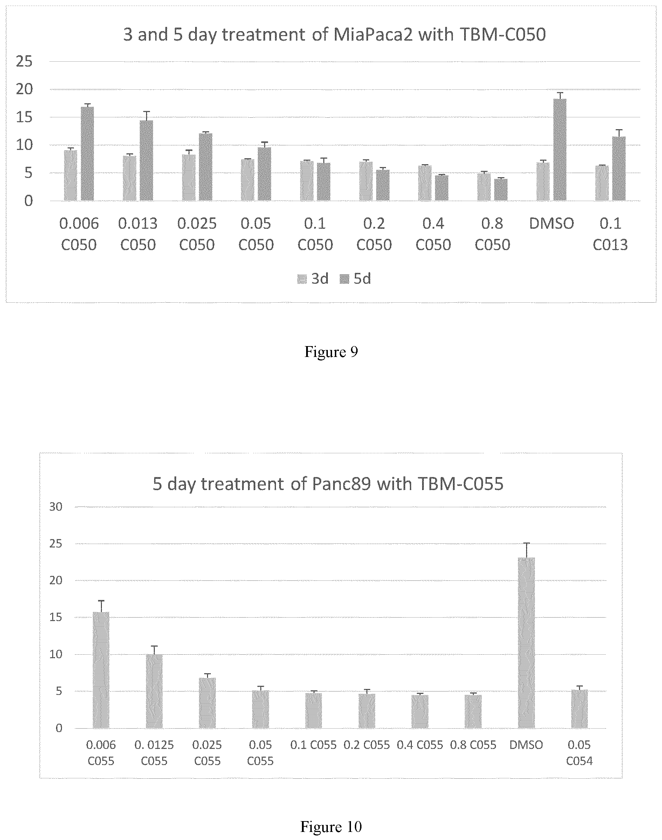

Figure 9 Inhibition of growth of PDAC line MiaPaca2 with [1,2,4] triazine derivative C050 ([1,2,4] triazine base outperforms adenine base (TBM-C013))
Figure 10 Efficient repression of Panc89 growth by the pegylated [1,2,4] triazine variant C055 (Efficient inhibition of Panc89 growth by pegylated C055)

A first aspect of the invention relates to a compound according to general formula (I) 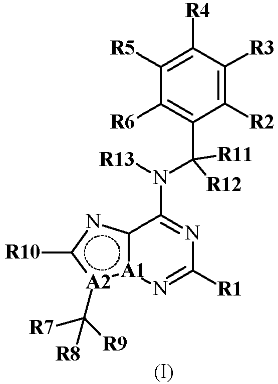 wherein
one of **A1** and **A2** means N and the other one of **A1** and **A2** means C;
**R1** means -F, -Cl, -NHC₁₋₆-alkyl, -NHC₁₋₆-alkylene-OH, -NHC₁₋₆-alkylene-NH₂, -(hetero-)cycloalkyl or -O-(hetero-)cycloalkyl;
   wherein -(hetero-)cycloalkyl or -O-(hetero-)cycloalkyl is optionally substituted with one, two or three substituents independently of one another selected from -F, -Cl, -CN, -C₁₋₆-alkyl, -C₁₋₆-alkylene-OH, -C₁₋₆-alkylene-CO₂H, -C₁₋₆-alkylene-C(=O)OC₁₋₆-alkyl, -C₁₋₆-alkylene-OC(=O)C₁₋₆-alkyl, -OH, -OC₁₋₆-alkyl, -OC₁₋₆-alkylene-OH, -O(CH₂CH₂O)ₙ-H with n = 1-50 (preferably 1-20), -OC(=O)C₁₋₆-alkyl, and -(hetero-)aryl;
   wherein -(hetero-)aryl is optionally substituted with one, two or three substituents independently of one another selected from -F, -Cl, -CN, -C₁₋₆-alkyl, -C₁₋₆-alkylene-OH, -C₁₋₆-alkylene-CO₂H, -C₁₋₆-alkylene-C(=O)OC₁₋₆-alkyl, -C₁₋₆-alkylene-OC(=O)C₁₋₆-alkyl, -OH, -OC₁₋₆-alkyl, -OC₁₋₆-alkylene-OH, -O(CH₂CH₂O)ₙ-H with n = 1-50 (preferably 1-20), and -OC(=O)C₁₋₆-alkyl;
**R2** means -N(C₁₋₆-alkyl)₂ or -(hetero-)aryl;
   wherein -(hetero-)aryl is optionally substituted with one, two or three substituents independently of one another selected from -F, -Cl, -CN, -C₁₋₆-alkyl, -C₁₋₆-alkylene-OH, -C₁₋₆-alkylene-CO₂H, -C₁₋₆-alkylene-C(=O)OC₁₋₆-alkyl, -C₁₋₆-alkylene-OC(=O)C₁₋₆-alkyl, -OH, -OC₁₋₆-alkyl, -OC₁₋₆-alkylene-OH, -O(CH₂CH₂O)ₙ-H with n = 1-50 (preferably 1-20), and -OC(=O)C₁₋₆-alkyl;
**R3, R4, R5, R6, R7, R8, R9, R10, R11** and **R12** independently of one another mean -H, -F, -Cl, -CN, -C₁₋₆-alkyl, -C₁₋₆-alkylene-OH, -C₁₋₆-alkylene-CO₂H, -C₁₋₆-alkylene-C(=O)OC₁₋₆-alkyl, -C₁₋₆-alkylene-OC(=O)C₁₋₆-alkyl, -OH, -OC₁₋₆-alkyl, -OC₁₋₆-alkylene-OH, -O(CH₂CH₂O)ₙ-H with n = 1-50 (preferably 1-20), and -OC(=O)C₁₋₆-alkyl;
or **R2** and **R3** together with the carbon atoms to which they are attached form a ring and mean -CH=CH-O-;
   and
**R13** means -H or -C₁₋₆-alkyl;
or a physiologically acceptable salt thereof.

In a preferred embodiment, **R1** means -(hetero-)cycloalkyl or -O-(hetero-)cycloalkyl; preferably -O-piperidinyl or -piperazinyl.

In a preferred embodiment, **R2** means -N(CH₃)₂ or -pyrazolyl.

In a preferred embodiment, the compound is of general formula (II-A) or (II-B) 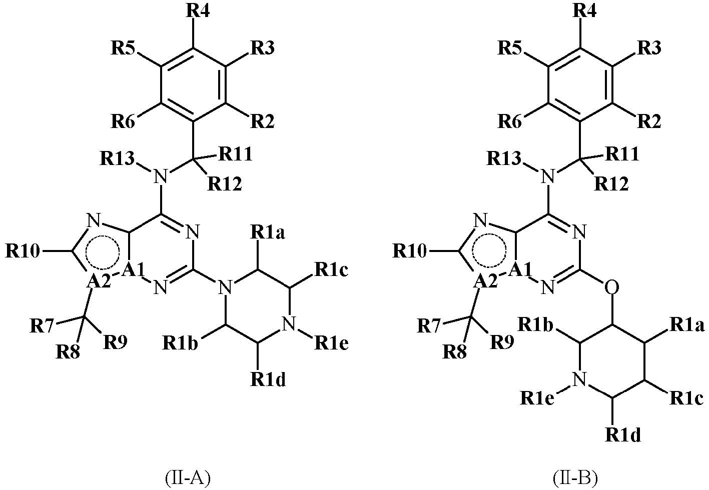 wherein
**R1a, R1b, R1c** and **R1d** independently of one another mean -H, -F, -Cl, -CN, -C₁₋₆-alkyl, -OH, -C₁₋₆-alkylene-OH, or -O-C₁₋₆-alkyl; and
**R1e** means -H or -C₁₋₆-alkyl.

In a preferred embodiment, **R1b** and **R1d** mean -H.

In a preferred embodiment, the compound is of general formula (III) 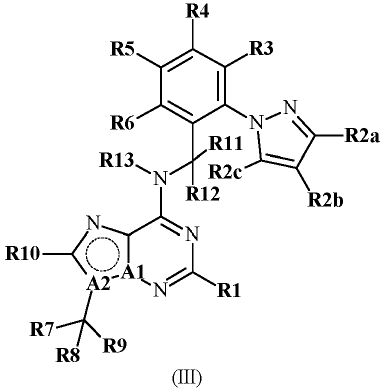 wherein
**R2a, R2b**, and **R2c** independently of one another mean -H, -F, -Cl, -CN, -C₁₋₆-alkyl, -OH, -C₁₋₆-alkylene-OH, or -O-C₁₋₆-alkyl.

In a preferred embodiment, the compound is of general formula (IV-A) or (IV-B) 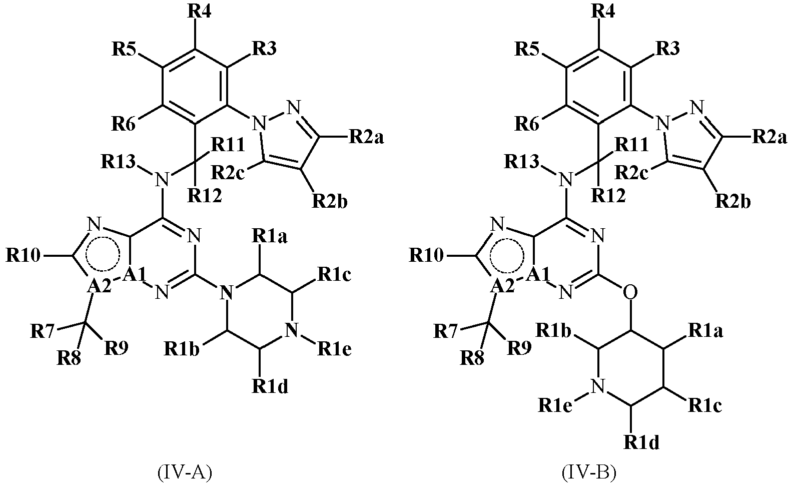 wherein
**R1a**, **R1b, R1c** and **R1d** independently of one another mean -H, -F, -Cl, -CN, -C₁₋₆-alkyl, -OH, -C₁₋₆-alkylene-OH, or -O-C₁₋₆-alkyl;
**R1e** means -H or -C₁₋₆-alkyl; and
**R2a, R2b,** and **R2c** independently of one another mean -H, -F, -Cl, -CN, -C₁₋₆-alkyl, -OH, -C₁₋₆-alkylene-OH, or -O-C₁₋₆-alkyl.

In a preferred embodiment, **R4** and **R6** mean -H.

In a preferred embodiment, **R5** means -H or -OH.

In a preferred embodiment, one of **R7, R8** and **R9** means -H and the other two of **R7, R8** and **R9** both mean -CH₃, -F, or -CH₂OH.

In a preferred embodiment, **R3** means -H, -C₁₋₆-alkylene-OH, -OH, -OC₁₋₆-alkyl, or -O(CH₂CH₂O)ₙ-H with n = 1-20; preferably -H, -CH₂CH₂OH, -OH, or -O(CH₂CH₂O)ₙ-H with n = 1-20.

In a preferred embodiment, **R10** means -H.

In a preferred embodiment, **R11** and **R12** mean -H.

In a preferred embodiment, the compound is selected from the group consisting of

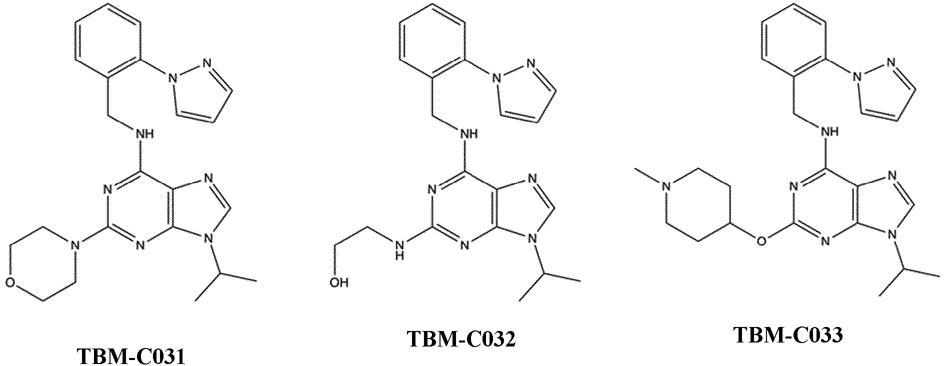

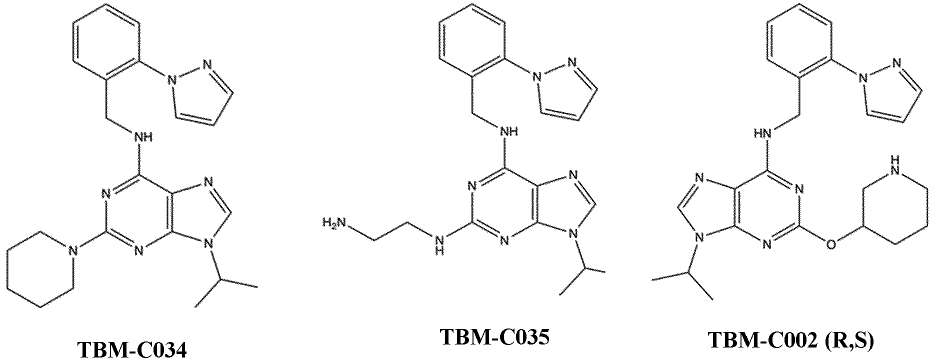

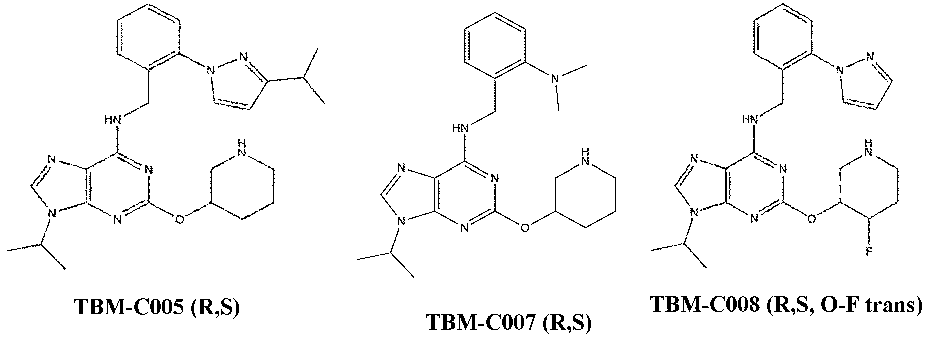

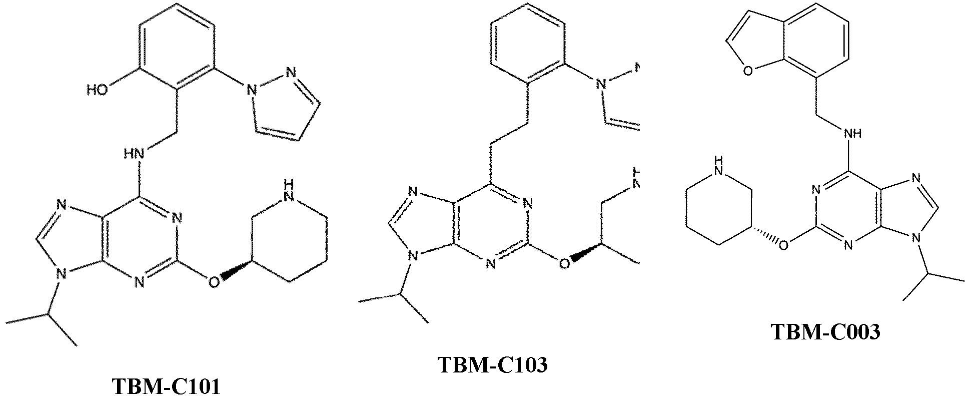

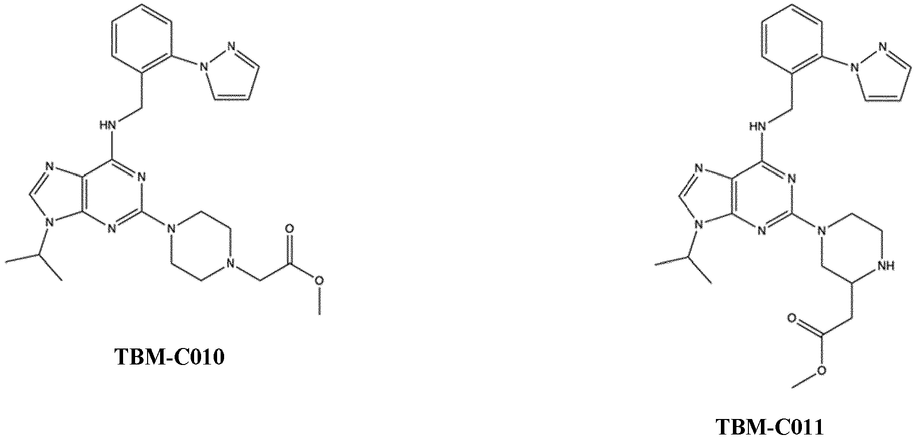

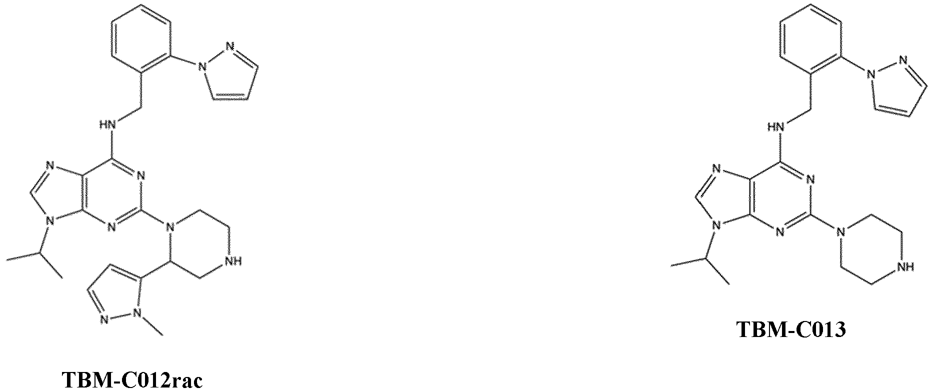

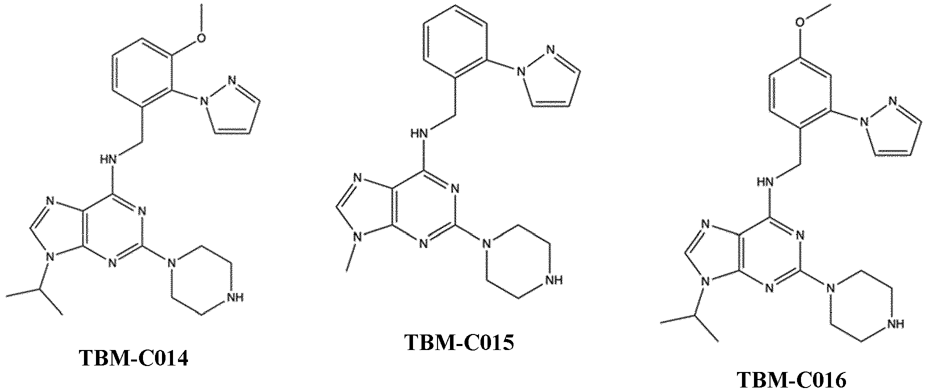

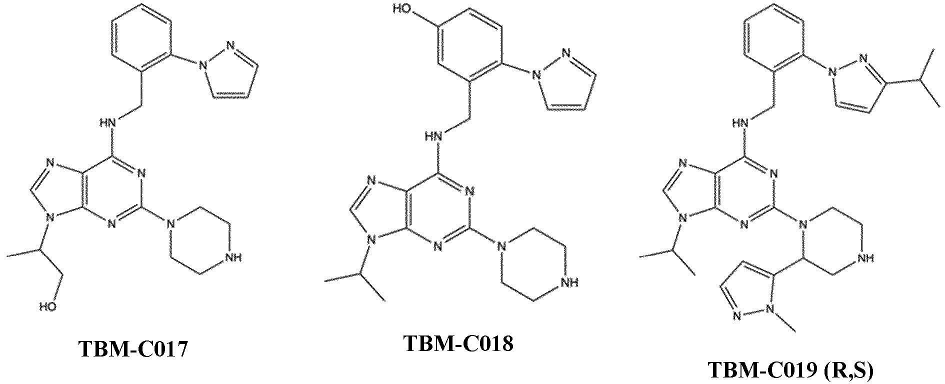

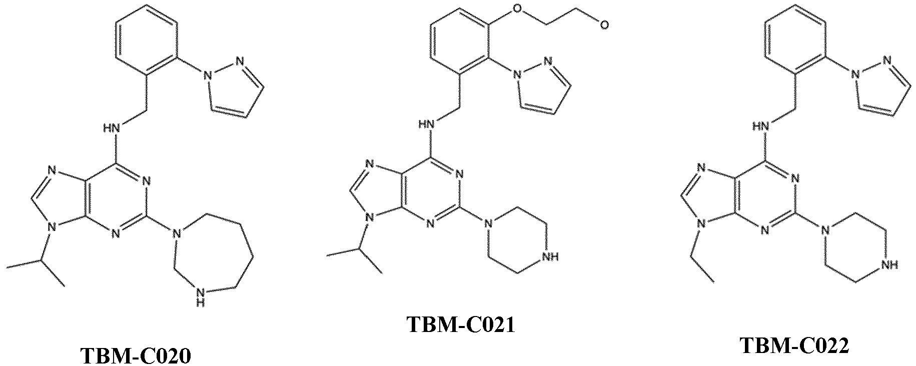

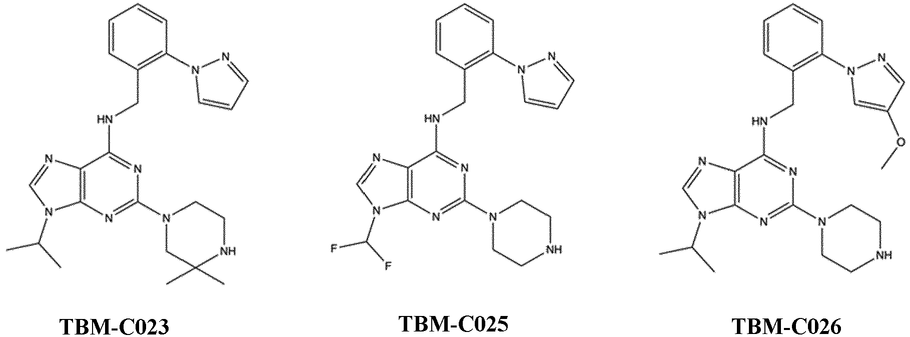

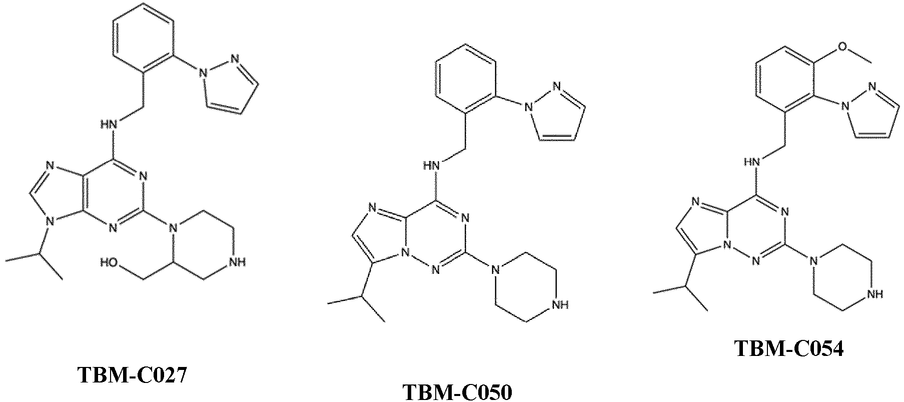

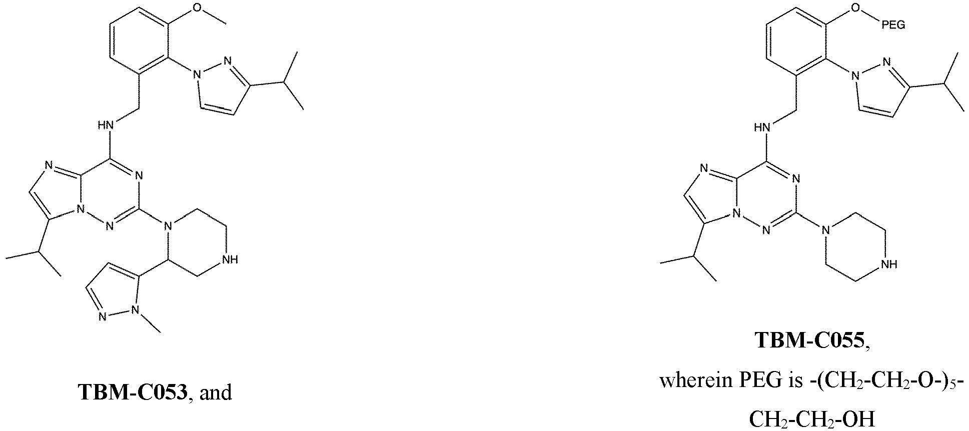

More preferably, the compound is selected from the group consisting of compound TBM-C013, compound TBM-C014, TBM-C019, compound TBM-C021 and a physiologically acceptable salt thereof.

Even more preferably, the compound is selected from the group consisting of compound TBM-C050, TBM-C053, compound TBM-C054 and a physiologically acceptable salt thereof.

TBM-C055 has the following structure:

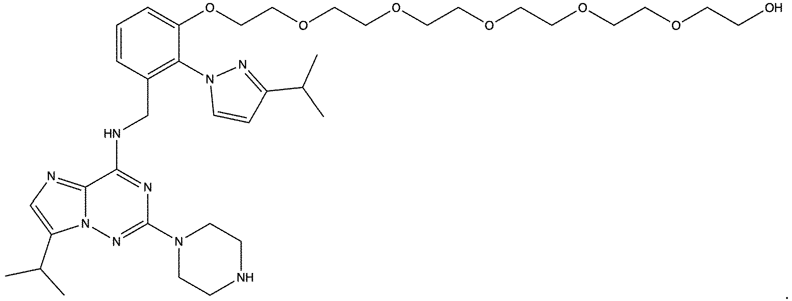

Preferably, unless otherwise stated, the following terms used in the specification and claims have the following meanings:

According to the invention, "-C₁₋₆-alkyl" and any other alkyl residues can be linear or branched, saturated or unsaturated. Linear saturated alkyl includes methyl, ethyl, n-propyl, n-butyl, n-pentyl and n-hexyl. Examples of branched saturated alkyl include but are not limited to iso-propyl, sec-butyl, and tert-butyl. Examples of linear unsaturated alkyl include but are not limited to vinyl, propenyl, allyl, and propargyl. In a preferred embodiment, "-C₁₋₆-alkyl" is saturated.

According to the invention, "-C₁₋₆-alkyl" and any other alkyl residues can be unsubstituted, mono- or polysubstituted. Examples of substituted alkyl include but are not limited to -CH₂CH₂OH, -CH₂CH₂OCH₃, -CH₂CH₂CH₂OCH₃, -CH₂CH₂S(=O)₂CH₃, -CH₂C(=O)NH₂, -C(CH₃)₂C(=O)NH₂, -CH₂C(CH₃)₂C(=O)NH₂, -CH₂CH₂C(=O)N(CH₃)₂, -C(=O)CH₃, -C(=O)C(CH₃)₃, -C(=O)-CH₂-CF₃, -C(=O)N(CH₃)₂, -C(=O)NH₂, and -S(=O)₂-CH₃. In a preferred embodiment, "-C₁₋₆-alkyl" is unsubstituted.

According to the invention, "cycloalkyl" means a non-aromatic, monocyclic, bicyclic or tricyclic moiety comprising 3 to 12 ring carbon atoms but no heteroatoms in the ring.

Examples of preferred saturated cycloalkyl moieties include but are not limited to cyclopropane, cyclobutane, cyclopentane, cyclohexane, cycloheptane, cyclooctane, hydrindane, and decaline. Examples of preferred unsaturated cycloalkyl moieties include but are not limited to cyclopropene, cyclobutene, cyclopentene, cyclopentadiene, cyclohexene, 1,3-cyclohexadiene, and 1,4-cyclohexadiene. In a preferred embodiment, "cycloalkyl" is saturated.

According to the invention, "heterocycloalkyl" means a non-aromatic, monocyclic, bicyclic or tricyclic moiety comprising 3 to 12 ring atoms, wherein each cycle comprises independently of one another 1, 2, 3, 4 or more heteroatoms independently of one another selected from the group consisting of nitrogen, oxygen and sulfur, whereas sulfur may be oxidized (S(=O) or (S(=O)₂), whereas the remaining ring atoms are carbon atoms, and whereas bicyclic or tricyclic systems may share common heteroatom(s).

Examples of preferred saturated heterocycloalkyl moieties include but are not limited to aziridin, azetidine, pyrrolidine, imidazolidine, pyrazolidine, piperidine, piperazine, triazolidine, tetrazolidine, oxiran, oxetane, tetrahydrofuran, tetrahydropyran, thiirane, thietane, tetrahydrothiophene, diazepane, oxazolidine, isoxazolidine, thiazolidine, isothiazolidine, thiadiazolidine, morpholine, thiomorpholine. Examples of preferred unsaturated heterocycloalkyl moieties include but are not limited to oxazoline, pyrazoline, imidazoline, isoxazoline, thiazoline, isothiazoline, and dihydropyran.

In association with "alkyl" or "-(hetero-)cycloalkyl", "mono- or polysubstituted" is preferably understood to mean the mono- or polysubstitution, e.g. the mono-, di-, or trisubstitution, of one or more hydrogen atoms by -F, -Cl, -CN, -C₁₋₆-alkyl, -C₁₋₆-alkyl-OH, -C₁₋₆-alkyl-CO₂H, -C₁₋₆-alkyl-C(=O)OC_{1 6}-alkyl, -C₁₋₆-alkyl-OC(=O)C₁₋₆-alkyl, -OH, -OC₁₋₆-alkyl, -OC₁₋₆-alkyl-OH, -O(CH₂CH₂O)ₙ-H with n = 1-20, -OC(=O)C₁₋₆-alkyl, or -(hetero-)aryl.

According to the invention, "-C₁₋₆-alkylene-" and any other alkylene residue can be unsubstituted, mono- or polysubstituted. Examples of saturated alkylene include but are not limited to -CH₂-, -CH(CH₃)-, -C(CH₃)₂-, -CH₂CH₂-, -CH(CH₃)CH₂-, -CH₂CH(CH₃)-, -CH(CH₃)-CH(CH₃)-, -C(CH₃)₂CH₂-, -CH₂C(CH₃)₂-, -CH(CH₃)C(CH₃)₂-, -C(CH₃)₂CH(CH₃)-, C(CH₃)₂C(CH₃)₂-, -CH₂CH₂CH₂-, and -C(CH₃)₂CH₂CH₂-. Examples of unsaturated alkylene include but are not limited to -CH=CH-, -C≡C-, -C(CH₃)=CH-, -CH=C(CH₃)-, -C(CH₃)=C(CH₃)-, -CH₂CH=CH-, -CH=CHCH₂-, -CH=CH-CH=CH-, and -CH=CH-C=C-. In a preferred embodiment, "-C₁₋₆-alkylene" is saturated.

According to the invention, "-C₁₋₆-alkylene-" and any other alkylene residue can be unsubstituted, mono- or polysubstituted. Examples of substituted -C₁₋₆-alkylene- include but are not limited to -CHF-, -CF₂-, -CHOH- -C(=O)-, and -C(=O)CH(NH₂)-. In a preferred embodiment, "-C₁₋₆-alkylene" is unsubstituted, except for those substituents which are defined in the claims.

According to the invention, "aryl", respectively independently, means a carbocyclic ring system with at least one aromatic ring, but without heteroatoms in this ring, wherein, if necessary, the aryl residues can be condensed with further saturated, (partially) unsaturated or aromatic ring systems, and each aryl residue can be present in unsubstituted or mono- or polysubstituted form, wherein the aryl substituents can be the same or different and in any desired and possible position of the aryl.

Preferred unsubstituted "aryls" are phenyl, naphthyl, anthracenyl, phenanthrenyl, fluoroanthenyl, fluoroenyl, indanyl and tetralinyl. Phenyl and naphthyl are particularly preferred. Preferred substituted "aryls" are 2-fluorophenyl, 3-fluorophenyl, 4-fluorophenyl, 2,3-difluorophenyl, 2,4-difluorophenyl, 3,4-difluorophenyl, 2-chlorophenyl, 3-chlorophenyl, 4-chlorophenyl, 2,3-dichlorophenyl, 2,4-dichlorophenyl, 3,4-dichlorophenyl, 2-methoxy-phenyl, 3-methoxy-phenyl, 4-methoxy-phenyl, 2,3-dimethoxy-phenyl, 2,4-dimethoxy-phenyl, 3,4-dimethoxy-phenyl, 2-methyl-phenyl, 3-methyl-phenyl, 4-methyl-phenyl, 2,3-dimethyl-phenyl, 2,4-dimethyl-phenyl and 3,4-dimethyl-phenyl, wherein the bonding can occur via any desirable and possible ring member of the aryl residue.

According to the invention, "heteroaryl", respectively independently, means a 5-, 6- or 7-membered cyclic aromatic residue, which contains 1, 2, 3, 4 or 5 heteroatoms, wherein the heteroatoms, the same or different, are nitrogen, oxygen or sulphur, and the heterocycle can be unsubstituted or mono- or polysubstituted; wherein in the case of the substitution on the heterocycle the substituents can be the same or different and can be in any desired and possible position of the heteroaryl; and wherein the heterocycle can also be part of a bi- or polycyclic system.

"Heteroaryl" is preferably selected from the group comprising pyrrolyl, indolyl, furyl (furanyl), benzofuranyl, thienyl (thiophenyl), benzothienyl, benzothiadiazolyl, benzooxadiazolyl, benzothiazolyl, benzooxazolyl, benzotriazolyl, benzodioxolanyl, benzodioxanyl, phthalazinyl, pyrazolyl, imidazolyl, thiazolyl, oxazolyl, isoxazoyl, pyridinyl, pyridazinyl, pyrimidinyl, pyrazinyl, pyranyl, indazolyl, purinyl, indolizinyl, quinolinyl, isoquinolinyl, quinazolinyl, carbazolyl, phenazinyl, phenothiazinyl or oxadiazolyl, wherein the bonding can occur via any desirable and possible ring member of the heteroaryl residue.

In association with "aryl" and "heteroaryl", "mono- or polysubstituted", respectively independently, means the mono- or polysubstitution of one or more hydrogen atoms of the ring system by substituents selected from the group comprising -F, -Cl, -CN, -C₁₋₆-alkyl, -C₁₋₆-alkyl-OH, -C₁₋₆-alkyl-CO₂H, -C₁₋₆-alkyl-C(=O)OC₁₋₆-alkyl, -C₁₋₆-alkyl-OC(=O)C₁₋₆-alkyl, -OH, -OC₁₋₆-alkyl, -OC₁₋₆-alkyl-OH, -O(CH₂CH₂O)ₙ-H with n = 1-20, and -OC(=O)C₁₋₆-alkyl.

The compounds according to the invention can be present in the form of a single stereoisomer or mixture thereof, the free compounds, their physiologically compatible salts and/or solvates.

The invention also includes isotopic isomers of a compound according to the invention, wherein at least one atom of the compound is replaced by an isotope of the respective atom which is different from the naturally predominantly occurring isotope, as well as any mixtures of isotopic isomers of such a compound. Preferred isotopes are ²H (deuterium), ³H (tritium), ¹³C and ¹⁴C.

The compounds according to the invention can be chiral or achiral, depending on the substitution pattern.

If the compounds according to the invention are chiral, then they are preferably present as racemate or a mixture of stereoisomers or diastereomers or in enriched form of an enantiomer. In a preferred embodiment the enantiomer excess (ee) of the S-enantiomer amounts to at least 50% ee, more preferred at least 75% ee, more preferred at least 90% ee, most preferred at least 95% ee, and in particular at least 99% ee. In another preferred embodiment, the enantiomer excess (ee) of the R-enantiomer amounts to at least 50% ee, more preferred at least 75% ee, more preferred at least 90% ee, most preferred at least 95% ee, and in particular at least 99% ee.

Suitable methods for separating the enantiomers are known to the person skilled in the art. Preparative HPLC on chiral stationary phases and conversion into diastereomeric intermediates can be given as examples. The conversion into diastereomeric intermediates can occur, for example, as salt formation by means of chiral, enantiomer-pure acids. After separation of the diastereomers thus formed, the salt can then be converted into the free base or another salt again.

Unless expressly specified, each reference to the compounds according to the invention covers all isomers in pure form and admixture with one another (e.g. stereoisomers, diastereomers, enantiomers) in any desired mixture ratio.

Unless expressly specified, each reference to the compounds according to the invention covers the free compounds (i.e. the forms that are not present in the form of salt) and all physiologically compatible salts.

For the purposes of the description, physiologically compatible salts of the compounds according to the invention are present as salts with anions or acids of the respective compound with inorganic or organic acids, which are physiologically compatible - in particular on application in humans and/or mammals.

Examples of physiologically compatible salts of specific acids are salts of: hydrochloric acid, hydrobromic acid, sulphuric acid, methane sulphonic acid, formic acid, acetic acid, oxalic acid, succinic acid, malic acid, tartaric acid, mandelic acid, fumaric acid, lactic acid, citric acid, glutamic acid, saccharinic acid, monomethyl sebacic acid, 5-oxo-proline, hexane-1-sulphonic acid, nicotinic acid, 2-, 3- or 4-aminobenzoic acid, 2,4,6-trimethyl benzoic acid, α-liponic acid, acetylglycine, acetylsalicylic acid, hippuric acid and/or aspartic acid. The hydrochloride, citrate and hemicitrate are particularly preferred.

Physiologically compatible salts with cations or bases are salts of the respective compound - as anion with at least one, preferably inorganic, cation, which are physiologically compatible - in particular on application in humans and/or mammals. Particularly preferred are the salts of the alkali and earth alkali metals, also ammonium salts, but in particular (mono-) or (di-) sodium, (mono-) or (di-) potassium, magnesium or calcium salts.

Another aspect of the invention relates to the compounds according to the invention as described above as medicaments. The amount of the compounds according to the invention to be administered to the patient varies in dependence on the weight of the patient, on the type of administration, on the indication and on the severity of the disease. Usually, from 0.00005 mg/kg to 50 mg/kg, preferably from 0.001 mg/kg to 10 mg/kg, of at least one compound according to the invention is administered.

Another aspect of the invention relates to pharmaceutical compositions or pharmaceutical dosage forms comprising the compounds according to the invention as described above. The pharmaceutical composition or pharmaceutical dosage form according to the invention contains the compound according to the invention in an amount of preferably from 0.001 to 99 wt. %, more preferably from 0.1 to 90 wt. %, yet more preferably from 0.5 to 80 wt. %, most preferably from 1.0 to 70 wt. % and in particular from 2.5 to 60 wt. %, based on the total weight of the pharmaceutical composition.

Preferably, the pharmaceutical compositions comprise a compound according to the invention as described above, and a pharmaceutically acceptable carrier. The term "pharmaceutically acceptable carrier" as used herein, means a non-toxic, inert solid, semi-solid or liquid filler, diluent, encapsulating material or formulation auxiliary of any type.

Some examples of materials which may serve as pharmaceutically acceptable carriers are sugars such as lactose, glucose and sucrose; starches such as com starch and potato starch; cellulose and its derivatives such as sodium carboxymethyl cellulose, ethyl cellulose and cellulose acetate; powdered tragacanth; malt; gelatin; talc; cocoa butter and suppository waxes; oils such as peanut oil, cottonseed oil, safflower oil, sesame oil, olive oil, corn oil and soybean oil; glycols such a propylene glycol; esters such as ethyl oleate and ethyl laurate; agar; buffering agents; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as releasing agents, coloring agents, coating agents, sweetening, flavoring and perfuming agents, preservatives and antioxidants may also be present in the composition, according to the judgment of one skilled in the art of formulations.

The pharmaceutical compositions may be administered to subjects (e.g., humans and other mammals) orally, rectally, parenterally, intravaginally, intracisternally, intraperitoneally, topically (as by powders, ointments or drops), bucally, extracorporeally, e.g. by dialysis, or as an oral or nasal spray. The term "parenterally" as used herein, refers to modes of administration, including intravenous, intramuscular, intraperitoneal, intrasternal, subcutaneous, intraarticular injection and infusion.

Pharmaceutical compositions for parenteral injection comprise pharmaceutically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (polyethylene glycol, propylene glycol, glycerol, and the like, and suitable mixtures thereof), vegetable oils (such as olive oil) and injectable organic esters such as ethyl oleate, or suitable mixtures thereof. Suitable fluidity of the composition may be maintained, for example, by the use of a coating such as lecithin, by the maintenance of the required particle size in the case of dispersions, and by the use of surfactants.

These compositions may also contain adjuvants, such as preservative agents, wetting agents, emulsifying agents, and dispersing agents. Prevention of the action of microorganisms may be ensured by various antibacterial and antifungal agents, for example, parabens, phenol, chlorobutanol, sorbic acid, and the like. It also may be desirable to include isotonic agents, for example, sugars, sodium chloride and the like. Prolonged absorption of the injectable pharmaceutical form may be brought about by the use of agents delaying absorption, for example, aluminium monostearate and gelatin.

In some cases, in order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug may depend upon its rate of dissolution, which, in turn, may depend upon crystal size and crystalline form. Alternatively, a parenterally administered drug form may be administered by dissolving or suspending the drug in an oil vehicle.

Suspensions, in addition to the active compounds, may contain suspending agents, for example, polyoxyethylene sorbitol, ethoxylated isostearyl alcohols, and sorbitan esters, microcrystalline cellulose, aluminium metahydroxide, bentonite, agar-agar, tragacanth, and mixtures thereof.

If desired, and for more effective distribution, the compounds may be incorporated into slow-release or targeted-delivery systems such as polymer matrices, liposomes, and microspheres. They may be sterilized, for example, by filtration through a bacteria-retaining filter or by incorporation of sterilizing agents in the form of sterile solid compositions, which may be dissolved in sterile water or some other sterile injectable medium immediately before use.

Injectable depot forms are made by forming microencapsulated matrices of the drug in biodegradable polymers such as polylactide-polyglycolide. Depending upon the ratio of drug to polymer and the nature of the particular polymer employed, the rate of drug release may be controlled. Depot injectable formulations also are prepared by entrapping the drug in liposomes or microemulsions which are compatible with body tissues.

The injectable formulations may be sterilized, for example, by filtration through a bacterial-retaining filter or by incorporating sterilizing agents in the form of sterile solid compositions which may be dissolved or dispersed in sterile water or other sterile injectable medium just prior to use.

Injectable preparations, for example, sterile injectable aqueous or oleaginous suspensions may be formulated according to the known art using suitable dispersing or wetting agents, suspending agents and the like. The sterile injectable preparation also may be a sterile injectable solution, suspension or emulsion in a nontoxic, parenterally acceptable diluent or solvent such as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid are used in the preparation of injectables.

Solid dosage forms for oral administration include, but are not limited to, capsules, tablets, pills, powders, and granules. In such solid dosage forms, one or more compounds is mixed with at least one inert pharmaceutically acceptable carrier such as sodium citrate or dicalcium phosphate and/or a) fillers or extenders such as starches, lactose, sucrose, glucose, mannitol, and salicylic acid; b) binders such as carboxymethylcellulose, alginates, gelatine, polyvinylpyrrolidone, sucrose, and acacia; c) humectants such as glycerol; d) disintegrating agents such as agar-agar, calcium carbonate, potato or tapioca starch, alginic acid, certain silicates, and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay; and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using lactose or milk sugar as well as high molecular weight polyethylene glycols.

The solid dosage forms of tablets, dragees, capsules, pills, and granules may be prepared with coatings and shells such as enteric coatings and other coatings well-known in the pharmaceutical formulating art. They optionally may contain opacifying agents and also may be of a composition that they release the active ingredient(s) only, or preferentially, in a certain part of the intestinal tract in a delayed manner. Examples of materials useful for delaying release of the active agent may include polymeric substances and waxes.

Compositions for rectal or vaginal administration are preferably suppositories which may be prepared by mixing the compounds with suitable non-irritating carriers such as cocoa butter, polyethylene glycol or a suppository wax which are solid at ambient temperature but liquid at body temperature and therefore melt in the rectum or vaginal cavity and release the active compound.

Liquid dosage forms for oral administration may include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, iso-propyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions may also include adjuvants such as wetting agents, emulsifying and suspending agents, sweetening, flavoring, and perfuming agents.

Suspensions, in addition to the active compounds, may contain suspending agents, for example, ethoxylated isostearyl alcohols, polyoxyethylene sorbitol and sorbitan esters, microcrystalline cellulose, aluminum metahydroxide, bentonite, agar-agar, tragacanth, and mixtures thereof.

If desired, and for more effective distribution, the compounds may be incorporated into slow-release or targeted-delivery systems such as polymer matrices, liposomes, and microspheres. They may be sterilized, for example, by filtration through a bacteria-retaining filter or by incorporation of sterilizing agents in the form of sterile solid compositions, which may be dissolved in sterile water or some other sterile injectable medium immediately before use.

Dosage forms for topical or transdermal administration of a compound include ointments, pastes, creams, lotions, gels, powders, solutions, sprays, inhalants or patches. A desired compound is admixed under sterile conditions with a pharmaceutically acceptable carrier and any needed preservatives or buffers as may be required. Ophthalmic formulation, eardrops, eye ointments, powders and solutions are also contemplated as being within the scope of this disclosure.

The ointments, pastes, creams and gels may contain, in addition to an active compound, animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays may contain, in addition to the compounds, lactose, talc, silicic acid, aluminum hydroxide, calcium silicates and polyamide powder, or mixtures of these substances. Sprays additionally may contain customary propellants such as chlorofluorohydrocarbons.

Compounds also may be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes may be used.

The present compositions in liposome form may contain, in addition to the compounds, stabilizers, preservatives, and the like. The preferred lipids are the natural and synthetic phospholipids and phosphatidylcholines (lecithins) used separately or together. Methods to form liposomes are known in the art.

Dosage forms for topical administration of a compound according to the invention as described above include powders, sprays, ointments and inhalants. The active compound is mixed under sterile conditions with a pharmaceutically acceptable carrier and any needed preservatives, buffers or propellants. Ophthalmic formulations, eye ointments, powders and solutions are also possible. Aqueous liquid compositions may also be useful.

Another aspect of the invention relates to the compounds according to the invention as described above for use in the treatment or the prevention of an proliferative disease, preferably cancer, more preferably pancreatic cancer, p53-based cancer and/or cancer with high proliferation; spreading of a virus; inflammation; and/or sepsis.

Preferably, the cancer is selected from the group comprising or consisting of: adenocarcinoma, choroidal melanoma, acute leukemia, acoustic neurinoma, ampullary carcinoma, anal carcinoma, astrocytoma, basal cell carcinoma, pancreatic cancer, Desmoid tumor, bladder cancer, bronchial carcinoma, estrogen dependent and independent breast cancer, Burkitt's lymphoma, corpus cancer, Carcinoma unknown primary tumor (CUP-syndrome), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic tumor, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymphomas, stomach cancer, malignant melanoma, malignant neoplasia, malignant tumors gastrointestinal tract, breast carcinoma, rectal cancer, medulloblastomas, melanoma, meningiomas, Hodgkin's / Non-Hodgkin's lymphoma, mycosis fungoides, nasal cancer, neurinoma, neuroblastoma, kidney cancer, renal cell carcinomas, oligodendroglioma, esophageal carcinoma, osteolytic carcinomas and osteoplastic carcinomas, osteosarcomas, ovarian carcinoma, pancreatic carcinoma, penile cancer, plasmacytoma, prostate cancer, pharyngeal cancer, rectal carcinoma, retinoblastoma, vaginal cancer, thyroid carcinoma, esophageal cancer, T-cell lymphoma, thymoma, tube carcinoma, eye tumors, urethral cancer, urologic tumors, urothelial carcinoma, vulva cancer, wart appearance, soft tissue tumors, soft tissue sarcoma, Nephroblastoma, cervical carcinoma, tongue cancer, invasive ductal carcinoma, invasive lobular carcinoma, ductal carcinoma in situ, lobular carcinoma in situ, small-cell lung carcinoma, non-smallcell lung carcinoma, bronchial adenoma, pleuropulmonary blastoma, mesothelioma, brain stem glioma, hypothalamic glioma, cerebellar astrocytoma, cerebral astrocytoma, neuroectodermal tumor, pineal tumors, sarcoma of the uterus, salivary gland cancers, anal gland adenocarcinomas, mast cell tumors, pelvis tumor, ureter tumor, hereditary papillary renal cancers, sporadic papillary renal cancers, intraocular melanoma, hepatocellular carcinoma, cholangiocarcinoma, mixed hepatocellular cholangiocarcinoma, squamous cell carcinoma, malignant melanoma, Merkel cell skin cancer, non-melanoma skin cancer, hypopharyngeal cancer, nasopharyngeal cancer, oropharyngeal cancer, oral cavity cancer, squamous cell cancer, oral melanoma, AIDS- related lymphoma, cutaneous T-cell lymphoma, lymphoma of the central nervous system, malignant fibrous histiocytoma, lymph sarcoma, rhabdomyosarcoma, malignant histiocytosis, fibroblastic sarcoma, hemangiosarcoma, hemangiopericytoma, leiomyosarcoma (LMS), canine mammary carcinoma, and/or feline mammary carcinoma.

In a preferred embodiment, the compounds according to the invention are for administration once daily. In another preferred embodiment, the compounds or physiologically acceptable salts or pharmaceutical compositions or pharmaceutical dosage forms according to the invention are for administration multiple times daily, in particular twice daily or thrice daily, or more often or continuously infused.

In a preferred embodiment, the compounds according to the invention are administered orally. In another preferred embodiment, the compounds according to the invention are administered parenterally. In another preferred embodiment, the compounds according to the invention are administered systemically, locally or extracorporeally.

Another aspect of the invention relates to a process for the preparation of the compounds according to the invention.

The following examples further illustrate the invention but are not to be construed as limiting its scope:

### EXAMPLES

### Synthesis scheme for C054:

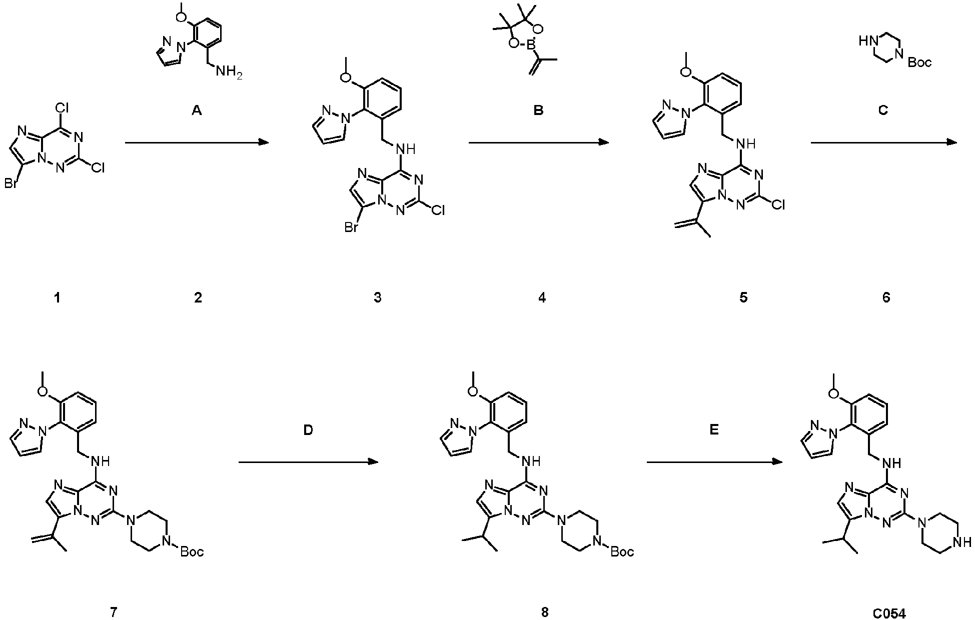

**Step A:** To a solution of compound **1** (6 g, 22.4 mmol, 1 eq.) in isopropanol (150 mL) was added 1-[3-methoxy-2-(1H-pyrazol-1-yl)phenyl]methanamine dihydrochloride 2 (9.3 g, 33 mmol, 1.5 eq.), DIPEA (23 mL, 0.135 mol, 5 eq.) and stirred at rt for 18 h. The reaction mixture was concentrated *in* vacuo. The residue was purified by FC (CyHex/EtOAc, 0-50%) to afford compound 3 (7.2 g, 16 mmol, 74%) as a white solid. LCMS purity >95%.

**Step B:** To a solution of compound **3** (7.2 g, 16 mmol, 1 eq.) in Dioxane (60 mL) and H₂O (15 ml) was added 4,4,5,5-tetramethyl-2-(prop-1-en-2-yl)-1,3,2-dioxaborolane (4) (8.35 g, 49.6 mmol, 3 eq.), Pd(dppf)Cl₂ (1.32 g, 1.6 mmol, 0.1 eq), Cs₂CO₃ (16.19 g, 49.6 mmol, 3 eq.) and stirred under N2 at 100 °C for 18 h. The reaction mixture was filtered through a pad of Celite^{®} washing with EtOAc and the filtrate was concentrated *in vacuo.* The residue was purified by FC (CyHex/EtOAc, 0-60%) to afford compound **5** (2.2 g, 4 mmol, 78%) was obtained as a white solid. LCMS purity >95%.

**Step C:** To a solution of compound **5** (5.4 g, 22.4 mmol, 1 eq.) in NMP (30 mL) was added tert-butyl piperazine-1-carboxylate **6** (12.7 g, 68 mmol, 5 eq.), DIPEA (12 mL, 68 mmol, 5 eq.) and stirred at rt for 18 h. The reaction mixture was diluted with EtOAc and washed with water (3x), brine, dried over Na₂SO₄ and concentrated *in vacuo.* The residue was purified by FC (CyHex/EtOAc, 0-60%) to afford compound **7** (2.8 g, 5.11 mmol, 37%) as a white solid. LCMS purity >95%.

**Step D:** To solution of compound 7 (2.8 mg, 5.13 mmol, 1 eq.) in MeOH (50 mL) was added Pd(OH)₂ (71 mg, 0.51 mmol, 0.1 eq.) and ammonium formate (3.28 g, 51 mmol, 10 eq.) and stirred at 70°C for 18 h. The reaction mixture was filtered through a pad of Celite^{®} washing with MeOH and the filtrate was concentrated *in vacuo.* The residue was purified by FC (C18, H₂O/MeOH, 0-100%) to afford compound **8** (2.2 g, 4 mmol, 78%) was obtained as a white solid. LCMS purity >95%.

**Step E:** To as solution of compound **8** (2.2 g 1 eq.) in Dioxane (40 mL) was added Dioxane/HCl (4 mL, 4 mol/l) and stirred at room temperature for 4 h. The reaction mixture was filtered, precipitate was washed MTBE, dried *in vacuo.* Afford the title compound **C054** (1.26 mg, 0.28 mmol, 95%) as white solid as a hydrochloric acid salt. LCMS purity >95%. 1H NMR (400 MHz, DMSO-d₆) δ 8.6 (t, J = 6 Hz, 1H), 7.83 (d, J = 2.3 Hz, 1H), 7.73 (d, J = 1.7 Hz, 1H), 7.39 (t, J = 8.1 Hz, 1H), 7.21 - 6.93 (m, 3H), 6.5 (t, J = 2.1 Hz, 1H), 4.3 (d, J = 6.1 Hz, 2H), 3.74 (s, 3H), 3.41 - 3.36 (m, 4H), 3.24 - 3.12 (m, 1H), 2.78 - 2.62 (m, 4H), 2.08 (s, 0H), 1.29 (d, J = 6.9 Hz, 6H).

### Further synthesis schemes:

Synthesis was carried out following the scheme given below:
**Step A:** Diisopropylethylamine (9.0 mL, 51.9 mmol) was added to a suspension of **1** (2.00 g, 8.7 mmol) and **2** (hydrochloride salt, 2.18 g, 10.4 mmol) in isopropanol (100 mL) the reaction mixture was heated to 80 °C and stirred for 5 h. The solvent was evaporated and the residue was purified by flash column chromatography (silica, cyclohexane → cyclohexane:EtOAc 34% to cyclohexane:EtOAc 66%) providing 3 (2.70 g) as a colorless solid.

¹H NMR (400 MHz,DMSO-d₆) δ 8.63 - 8.54 (m, 1H), 8.25 (d, J = 39.5 Hz, 2H), 7.79 (d, J = 1 Hz, 1H), 7.51 - 7.35 (m, 4H), 6.55 (t, J = 2 Hz, 1H), 5.19 - 4.57 (m, 3H), 1.5 (d, J = 6.7 Hz, 6H)
**Step B:** A stirred solution of 3 (250 mg, 0.68 mmol) and the corresponding amine (1.5 equiv.) and K₂CO₃ in DMSO (2 mL) was stirred at 165 °C for 18 h. After the reaction was complete the reaction mixture was cooled down and poured into water. The aqueous phase was extracted with ethyl acetate (3x 50 mL), then the combined organic phase was washed with brine (50 mL). After column chromatography (C18-silica, water:MeCN 10:90 to MeCN) the products were obtained.

**3303-284 (C027):** off-white powder, ¹H NMR (400 MHz, DMSO-d₆) δ 8.12 (d, J = 2.1 Hz, 1H), 7.84 (s, 1H), 7.82 - 7.79 (m, 1H), 7.79 - 7.63 (m, 1H), 7.55 - 7.49 (m, 1H), 7.42 - 7.33 (m, 3H), 6.58 - 6.52 (m, 1H), 4.67 - 4.48 (m, 4H), 4.44 - 4.31 (m, 1H), 4.27 (s, 1H), 2.88 (d, J = 11.2 Hz, 1H), 2.69 - 2.59 (m, 1H), 2.59 - 2.51 (m, 3H), 2.39 - 2.3 (m, 1H), 1.45 (dd, J = 6.7, 1.3 Hz, 6H).

**3293-897 (C034):** off-white powder, ¹H NMR (400 MHz,DMSO-d₆) δ 8.12 (d, J = 2.1 Hz, 1H), 7.86 (s, 2H), 7.82 - 7.76 (m, 1H), 7.57 - 7.49 (m, 1H), 7.43 - 7.32 (m, 3H), 6.55 (t, J = 2.1 Hz, 1H), 4.65 - 4.48 (m, 3H), 3.56 (d, J = 4.2 Hz, 4H), 3.46 (s, 4H), 1.45 (d, J = 6.8 Hz, 6H).

**3303-227 (C032):** off-white powder, ¹H NMR (400 MHz,DMSO-d₆) δ 8.1 (d, J = 2.1 Hz, 1H), 7.84 - 7.66 (m, 3H), 7.56 - 7.5 (m, 1H), 7.42 - 7.32 (m, 3H), 6.55 (t, J = 2.1 Hz, 1H), 4.67 - 4.47 (m, 3H), 3.53 (s, 4H), 1.53 (d, J = 4.4 Hz, 2H), 1.45 (d, J = 6.8 Hz, 6H).

**3303-226 (C031):** off-white powder, ¹H NMR (400 MHz,DMSO-d₆) δ 8.12 (d, J = 2.1 Hz, 1H), 7.86 (s, 2H), 7.83 - 7.78 (m, 1H), 7.56 - 7.49 (m, 1H), 7.43 - 7.31 (m, 3H), 6.55 (t, J = 2.1 Hz, 1H), 4.65 - 4.48 (m, 3H), 3.56 (d, J = 4.2 Hz, 4H), 3.46 (s, 3H), 1.45 (d, J = 6.8 Hz, 6H).

**Step C: 3** (250 mg, 0.68 mmol) was added to ethylenediamine **8** (4.08 g, 100 equiv.) and the mixture was stirred at 70 °C for 18 h. The reaction mixture was then poured into water, extracted with ethyl acetate (3x 50 mL) and the combined organic phase was washed with brine (50 mL). After column chromatography (C18-silica, water:MeCN 10:90 to MeCN) **3303-225 (C035)** was obtained as an off white solid.

¹H NMR (400 MHz,DMSO-d₆) δ 8.16 (d, J = 2.3 Hz, 1H), 7.83 - 7.75 (m, 2H), 7.74 - 7.44 (m, 2H), 7.44 - 7.32 (m, 3H), 6.59 - 6.52 (m, 1H), 6.26 - 6.09 (m, 1H), 4.74 - 4.39 (m, 3H), 3.22 - 3.13 (m, 2H), 2.62 (s, 1H), 1.91 (s, 1H), 1.7 (s, 1H), 1.46 (d, J = 6.8 Hz, 6H).

**Step D:** To a solution of 9 (157 mg, 1.4 mmol, 2 equiv.) in DMF (3 mL) was added NaH (60% in mineral oil, 56 mg) and was stirred at room temperature for 30 minutes. Then **3** (250 mg, 0.68 mmol) was added and the resulting reaction mixture was stirred at 80 °C for 18 h. After completion of the reaction, it was poured into water, extracted with ethyl acetate (3x 50 mL) and the combined organic phase was washed with brine (50 mL). After column chromatography (C18-silica, water:MeCN 10:90 to MeCN) **3303-612 (C033)** was obtained as an brown solid.

¹H NMR (400 MHz,DMSO-d₆) δ 8.18 (d, J = 2.3 Hz, 2H), 8.04 (s, 1H), 7.8 (d, J = 1.5 Hz, 1H), 7.46 - 7.35 (m, 5H), 6.56 (t, J = 2.1 Hz, 1H), 4.81 - 4.52 (m, 4H), 3.29 (s, 3H), 2.82 - 2.63 (m, 2H), 2.36 - 2.31 (m, 2H), 1.91 (s, 2H), 1.69 - 1.57 (m, 2H), 1.48 (d, J = 6.8 Hz, 7H).

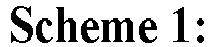

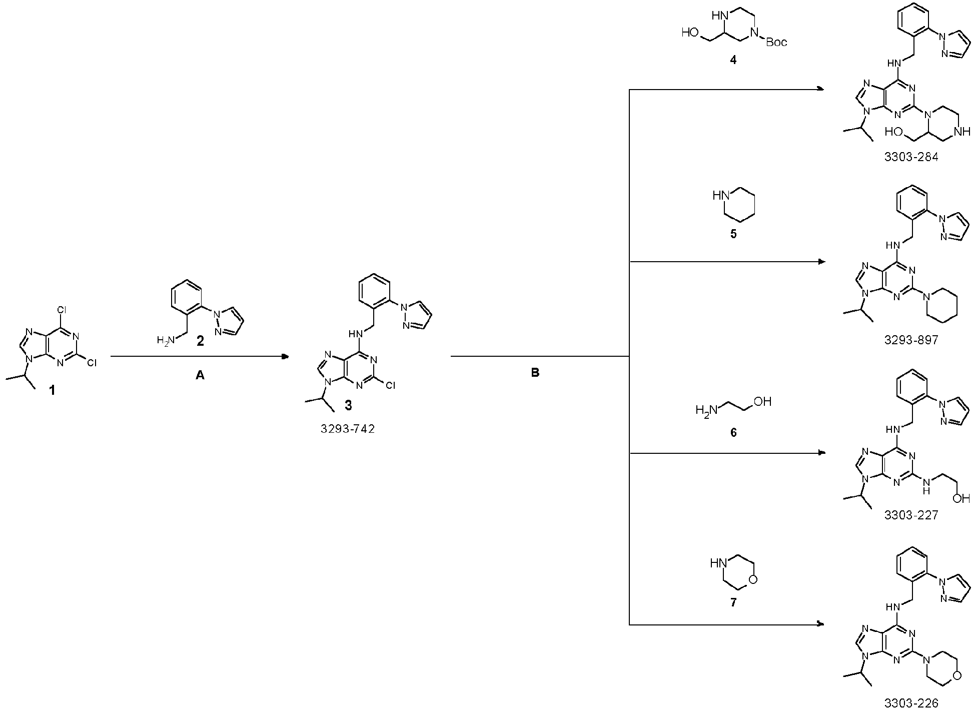

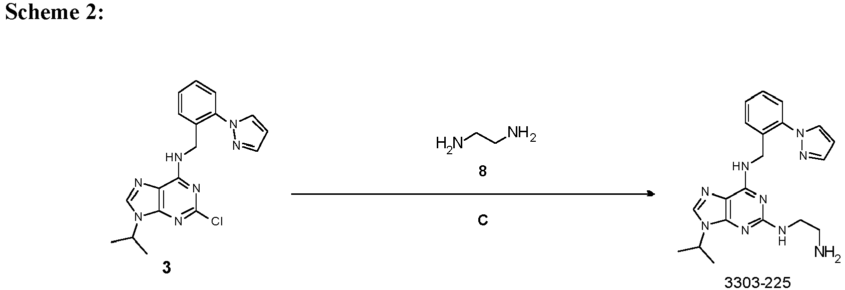

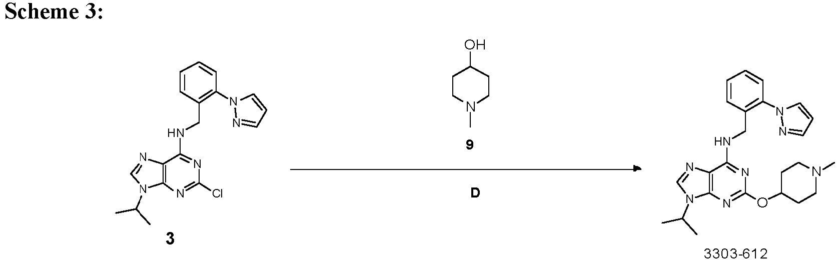

### Further synthesis schemes:

Synthesis was carried out following the scheme given below:

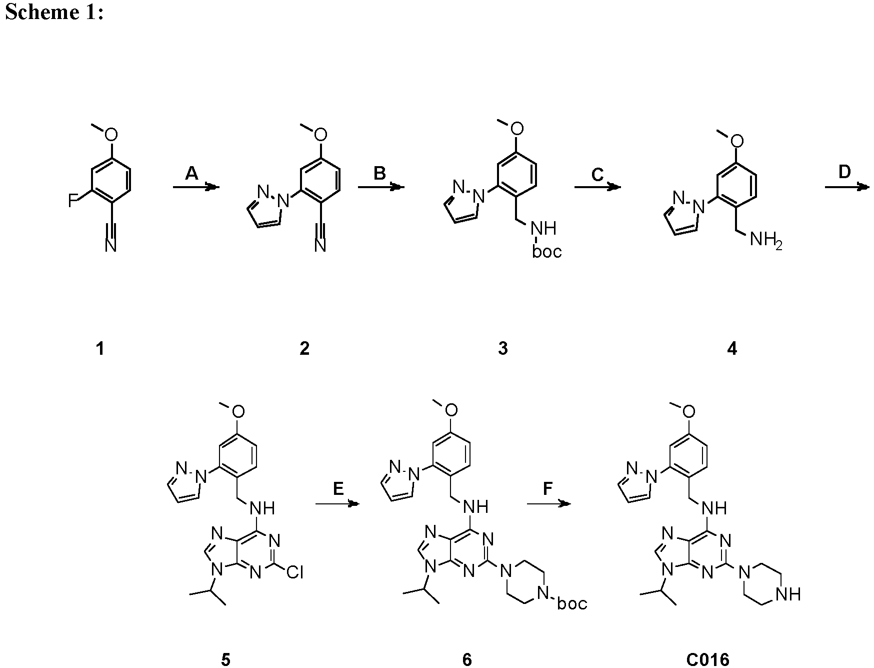

### Step A:

A flame-dried 2000 mL flask with a stir bar was charged pyrazole (31.28 g, 459.6 mmol), and the flask was degassed and filled with Ar, then DMF (750 mL) was added. The obtained mixture was cooled to 0°C, briefly opened to air, then NaH (19.3 g of a 60% w/w dispersion in mineral oil, 459.6 mmol) was added. The reaction mixture was stirred at RT for 15 min, then 2-fluoro-4-methoxybenzonitrile 1 (57.88 g, 383 mmol,) was added, and the obtained mixture was stirred at 60°C for 1 h, and then at RT for 16 h. The reaction was cooled to RT and quenched with sat. aq. NaHCO₃. The solution was extracted with EtOAc, and the organic fractions were combined, washed with H₂O and brine, dried over Na₂SO₄, and concentrated. The crude residue was triturated with IPA\hexane, filtered and washed with hexane to yield compound **2** (41 g, 54% yield).

### Step B:

A solution of compound **2** (9.8 g, 49.2 mmol), NiCl₂ · 6H₂O (1.17 g, 4.92 mmol) and di-tert-butyl dicarbonate (21.47 g, 98.4 mmol) in methanol was treated portionwise with NaBH₄ (9.3 g, 246 mmol) with stirring at 0°C and. The obtained mixture was stirred 3 h at RT and then concentrated under reduced pressure. The residue was diluted with water and extracted with DCM. The organic layer was dried over Na₂SO₄, and the solvent was evaporated. The residue was purified by flash column chromatography to obtain compound **3** (8.8 g, 59% yield).

### Step C:

A solution of compound **3** (8.8 g, 28.99 mmol) in DCM/Et₂O · HCl (120 mL) was stirred 2 h at RT. After completion of the reaction, the mixture was evaporated to dryness to obtain compound **4** (7.37 g) as HCl salt.

### Step D:

To a solution of 2,6-dichloro-9-(propan-2-yl)-9H-purine (6.38 g, 27.64 mmol) in *n*-BuOH (100 mL) compound **4** (6.95 g, 29.03 mmol) and TEA (9.8 g, 96.76 mmol) were added. After heating at 90 °C for 16 h, n-BuOH was evaporated in vacuo. After dilution with H₂O, the mixture was extracted with EtOAc and washed with water. The combined organic extracts were dried over Na₂SO₄ and the solvent was removed in vacuo to obtain compound **5** (8.56 g, 77% yield).

### Step E:

To a solution of compound 5 (7.26 g, 18.26 mmol) in NMP (30 mL) tert-butyl piperazine-1-carboxylate (3.74 g, 20.08mmol) and DIPEA (3.54 g, 27.38 mmol) were added, and the obtained mixture was stirred at 140 °C for 16 h. After completion of the reaction, the reaction mixture diluted with H₂O, extracted with EtOAc and washed with water. The combined organic extracts were dried over Na₂SO₄ and the solvent was removed in vacuo. The residue was purified by flash column chromatography to obtain compound 6 (2.3 g, 23% yield).

### Step F:

A solution of compound **6** (2.3 g, 4.24 mmol) in 10% mixture of TFAA in DCM (30 mL) was maintained in an ultrasonic bath at 20°C for 2 h. Then the volatiles were evaporated, and the residue was diluted with 10% KOH solution in H₂O, extracted with DCM and washed with water. The combined organic extracts were dried over Na₂SO₄ and the solvent was removed in vacuo to obtain compound **C016** (1.5 g, 75% yield, 95.17% purity).

MS Calcd.: 447.55 MS Found: 448.0 [M + H]⁺.

¹H NMR (500 MHz, dmso) δ 7.25 (t, *J =* 2.7 Hz, 1H), 7.16 (d, *J =* 2.7 Hz, 1H), 7.04 - 6.99 (m, 1H), 6.96 (d, *J =* 3.9 Hz, 1H), 6.32 (q, *J =* 3.3 Hz, 2H), 5.92 (q, *J* = 2.7 Hz, 1H), 5.87 (t*, J =* 5.9 Hz, 1H), 4.19 - 4.04 (m, 3H), 3.32 (t*, J* = 5.0 Hz, 4H), 3.26 (s, 3H), 2.46 (d, *J* = 9.9 Hz, 4H), 0.98 (d, *J =* 6.7 Hz, 6H).

Further synthesis schemes:

Synthesis was carried out following the scheme given below:

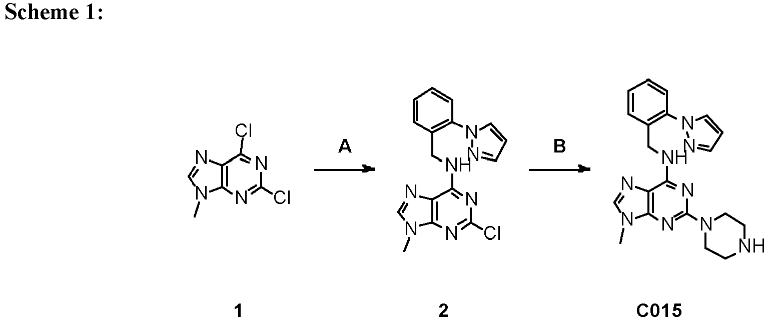

### Step A:

To a solution of [2-(1H-pyrazol-1-yl)phenyl]methanamine hydrochloride (9.72 g, 46.35 mmol) in butanol (150 mL) TEA (11.17 g, 110.36 mmol) and compound **1** (8.96 g, 44.15 mmol) were added, then the obtained mixture was stirred overnight at 90°C. The reaction mixture is evaporated, then poured with water and placed in a cold ultrasonic bath for 30 minutes. The formed precipitate was filtered, washed with water, IPS, hexane, and dried under vacuum to obtained compound 2 (11.2 g, 75% yield).

### Step B:

A mixture of compound **2** (2.78 g, 8.17 mmol) and tert-butyl piperazine-1-carboxylate (1.67 g, 8.99 mmol) was dissolved in DMA (30 mL), then to obtained solution DIPEA (1.58 g, 12.26 mmol) was added. Then the obtained mixture was stirred overnight at 140°C. The reaction mixture was cooled to RT, poured with water and extracted with EtOAc. The organic layer was washed with water, dried over Na₂SO₄ and evaporated.

The obtained Boc-product was dissolved in 100 mL of 20% TFA in DCM and left to stir at RT for 3 hours, then the reaction mixture was evaporated, poured with 10% KOH and extracted with DCM. The organic layer is washed with water, dried over Na₂SO₄, and evaporated. The obtained crude material was purified by prep-HPLC to obtained target compound C015 (2.3 g, 72% yeild, 95.17% purity).

MS Calcd.: 389.47 MS Found: 390.2 [M + H]⁺.

¹H NMR (500 MHz, cdcl₃) δ 7.80 (d, *J =* 1.8 Hz, 1H), 7.68 (dd, *J* = 14.4, 4.6 Hz, 2H), 7.40 (s, 1H), 7.37 - 7.28 (m, *J* = 4.4 Hz, 3H), 6.49 - 6.44 (m, 2H), 4.73 (s, 2H), 3.80 (t, *J =* 5.0 Hz, 4H), 3.63 (s, 3H), 2.94 (t, *J =* 5.0 Hz, 4H).

### Further synthesis schemes:

Synthesis of C013-OH (C018) was carried out following the scheme given below:

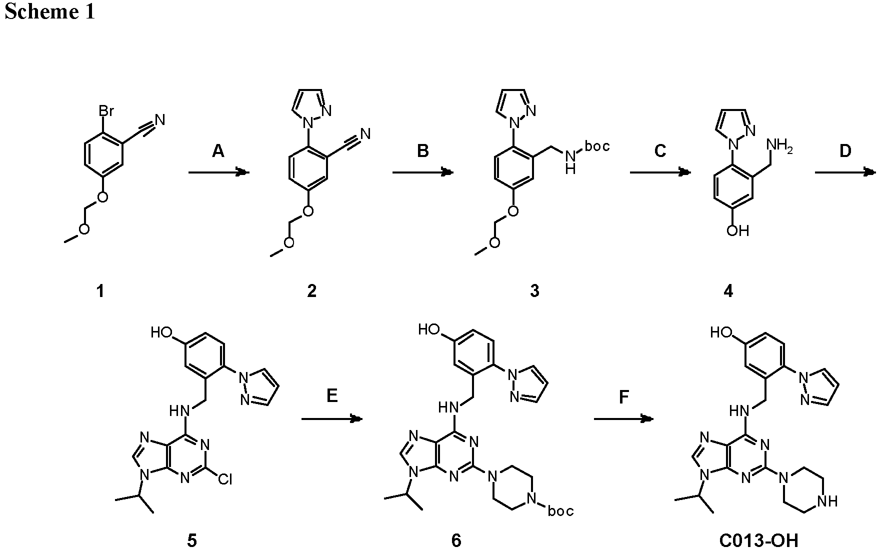

**Step A:** A mixture of compound 1 (2.1 g, 8.7 mmol), pyrazole (0.89 g, 13.1 mmol), Cs₂CO₃ (4.26 g, 13.1 mmol), N1,N2-dimethylcyclohexane-1,2-diamine (0.25 g, 1.75 mmol) and CuI (0.2 g, 0.87 mmol) in DMA (15 mL) was degassed and stirred at 120°C for 16 h. After the reaction completed, the mixture was cooled down to RT, diluted with water and extracted with EtOAc. The organic layer was washed with water, brine, dried and evaporated in vacuo. The crude material was purified by flash column chromatography to give compound **2** (0.31 g, 16% yield).

**Step B:** To a solution of compound **2** (0.31 g, 1.3 mmol), Boc₂O (0.59 g, 2.7 mmol) and NiCl₂ × 6 H₂O (32 mg, 0.14 mmol) in MeOH (50 mL) NaBH₄ (0.26 g, 6.7 mmol) was added portionwise under ice cooling. After completion of addition the mixture was allowed to warm up to RT and stir overnight. The mixture was concentrated in vacuo, the residue was diluted with aq. NH₄OH, stirred for 10 min and extracted with EtOAc. The organic layer was washed with water, brine, dried and evaporated in vacuo to give compound **2** (0.37 g, 80% purity). The reaction mixture was used in the next step without additional purification.

**Step C:** Compound **2** (0.37 g, 80% purity) was dissolved in DCM (20 mL) and 10 mL of HCl in ether was added. The reaction mixture was stirred at RT overnight, then evaporated to dryness to afford compound **4** (0.28 g 2 HCl, 80% yield over 2 steps).

**Step D:** A mixture of compound **4** (2 HCl, 0.28 g, 1.07 mmol), 3-(chloromethyl)-4-(1*H*-pyrazol-1-yl)phenol (0.25 g, 1.07 mmol) and DIPEA (0.456 g, 3.5 mmol) in NMP (3 mL) was stirred at 80°C for 16 h. The reaction mixture was used in the next step without isolation.

**Step E:** To the reaction mixture was added *tert*-butyl piperazine-1-carboxylate (0.54 g, 2.9 mmol) and DIPEA (0.3 g 2.3 mmol) and the reaction mixture was stirred for further 16 h at 140°C. After the reaction completed, the mixture was cooled down to RT and poured into water. The precipitated solid was collected washed with water and dried to afford crude compound **6** (0.4 g), which was used in the next step without purification.

**Step F:** Compound **6** (0.4 g) was dissolved in DCM (10 mL), and 10 mL of HCl in ether was added. The reaction mixture was stirred at RT overnight, then evaporated to dryness. The crude material was purified by prep-HPLC to give the target compound **C013-OH (C018)** (94 mg, 19% yield over 3 steps). MS Calcd.: 433.2 MS Found: 434.2 [M + H]⁺.

¹H NMR (500 MHz, dmso) δ 9.62 (s, 1H), 7.93 (s, 1H), 7.82 (s, 1H), 7.69 (d, *J =* 1.8 Hz, 2H), 7.14 (d, *J =* 8.5 Hz, 1H), 6.86 (d, *J =* 2.7 Hz, 1H), 6.67 (dd, *J* = 8.5, 2.8 Hz, 1H), 6.46 (t, *J* = 2.1 Hz, 1H), 4.54 (p, *J =* 6.7 Hz, 1H), 4.38 (s, 2H), 3.46 (s, 4H), 2.66 (s, 4H), 1.44 (d, *J* = 6.8 Hz, 6H).

### Further synthesis schemes:

Synthesis of TBM-C021 was carried out following the scheme given below:

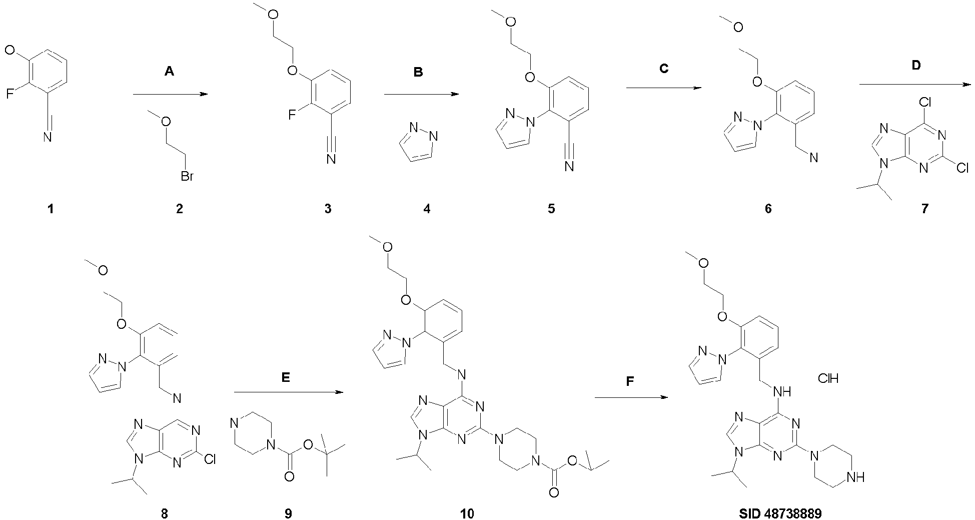

**Step A:** To a solution of 2-fluoro-3-hydroxybenzonitrile **(1)** (100 mg, 0.73 mmol, 1 eq.) in DMF (0.5 mL) was added K₂CO₃ (202 mg, 1.46 mmol, 2 eq.) and 1-bromo-2-methoxyethane **(2)** (82.2 µL, 0.88 mmol, 1.2 eq.) and stirred at 80 °C for 2 h. The resulting residue was taken up in EtOAc (10 mL) and washed with water (20 mL), brine (20 mL), dried over Na₂SO₄ and concentrated *in vacuo.* 2-Fluoro-3-(2-methoxyethoxy)benzonitrile **(3)** (139 mg, 0.72 mmol, 98%) was obtained as a colourless oil which was used without further purification. LCMS purity >99%.

**Step B:** To a solution of compound 3 (140 mg, 0.72 mmol, 1 eq.) in DMF (2 mL) was added pyrazole **(4)** (59 mg, 0.86 mmol, 1.2 eq.) and K₂CO₃ (198 mg, 1.43 mmol, 2 eq.) and stirred at 110 °C for 4 h. The reaction mixture was concentrated *in* vacuo. The resulting residue was taken up in EtOAc (10 mL) and washed with water (20 mL), brine (20 mL), dried over Na₂SO₄ and concentrated *in vacuo.* 3-(2-Methoxyethoxy)-2-(1*H*-pyrazol-1-yl)benzonitrile **(5)** (175 mg, 0.72 mmol, quant.) was obtained as a colourless oil which was used without further purification. LCMS purity >90%.

**Step C:** To solution of compound **5** (80 mg, 0.33 mmol, 1 eq.) in THF (8 mL) was added LiAlH₄ (37 mg, 0.99 mmol, 3 eq.) and stirred at room temperature for 1 h. The reaction mixture was quenched with water (0.1 mL), 4 M NaOH (0.2 mL) and stirred for 1 h, dried over Na₂SO₄ and concentrated *in vacuo.* 1-[3-(2-Methoxyethoxy)-2-(1*H*-pyrazol-1-yl)phenyl]methanamine **(6)** (53 mg, 0.21 mmol, 65%) was obtained as a colourless oil which was used without further purification. LCMS purity >95%.

**Step D:** To as solution of compound **6** (53 mg, 0.21 mmol, 1 eq.) in iPrOH (3 mL) was added 2,6-dichloro-9-(propan-2-yl)-9*H*-purine **(7)** (50 mg, 0.21 mmol, 1 eq.) and DIPEA (0.11 mL, 0.64 mmol, 3 eq.) and stirred at 80 °C for 18 h. The reaction mixture concentrated *in vacuo* and recrystallized with EtOAc to yield 2-chloro-*N*-{[3-(2-methoxyethoxy)-2-(1*H*-pyrazol-1-yl)phenyl]methyl}-9-(propan-2-yl)-9*H*-purin-6-amine **(8)** (35 mg, 0.078 mmol, 37%) as a white solid. LCMS purity >98%.

**Step E:** To a solution of compound **8** (35 mg, 0.078 mmol, 1 eq.) in NMP (1 mL) was added tert-butyl piperazine-1-carboxylate **(9)** (22 mg, 0.12 mmol, 1.5 eq.) and DIPEA (27.6 µL, 0.16 mmol, 2 eq.) and stirred at 140 °C for 40 h. The reaction mixture was directly purified by FC(MeOH:DCM, 0-2.5%) to afford *tert*-butyl 4-[6-({|3-(2-methoxyethoxy)-2-(1*H*-pyrazol-1-yl)phenyl|methyl}amino)-9-(propan-2-yl)-9H-purin-2-yl]piperazine-1-carboxylate **(10)** (34 mg, 0.057 mmol, 73%) as a brown oil. LCMS purity >99%.

**Step F:** To a solution of compound **10** (30 mg, 0.051 mmol. 1 eq.) in DCM (0.5 mL) was added 4 M HCl in dioxane (0.25 mL) and stirred at room temperature for 16 h. The reaction mixture was concentrated *in vacuo.* The resulting solid was taken up in cyclohexane, sonicated and dried to completeness to afford the title compound **C021** (24 mg, 0.045 mmol, 90%) as a beige solid as a hydrochloride salt. ¹H NMR (400 MHz, DMSO-d₆) δ 9.76 (1H, br s, NH), 9.28 (2H, br s, NH₂), 7.92 (1H, s, ArH), 7.86 (1H, d, *J =* 2.0 Hz, ArH), 7.74 (1H, d, *J* = 2.4 Hz, ArH), 7.33 (1H, t, *J* = 8.0 Hz, ArH), 7.09 (1H, d, *J* = 8.0 Hz, ArH), 7.03 (1H, d, *J* = 8.0 Hz, ArH), 6.50 (1H, t, *J =* 2.0 Hz, ArH), 4.58 (1H, quint, *J* = 6.8 Hz, CH), 4.28 (2H, br s, CH₂), 4.10 - 4.08 (2H, m, CH₂), 3.80 (4H, br s, 2 × CH₂), 3.53 - 3.51 (2H, m, CH₂), 3.19 (3H, s, OMe), 3.04 (4H, br s, 2 × CH₂), 1.46 (6H, d, *J =* 6.7 Hz, , 2 × Me)

### Further synthesis schemes:

Synthesis of C002-S was carried out following the scheme given below:

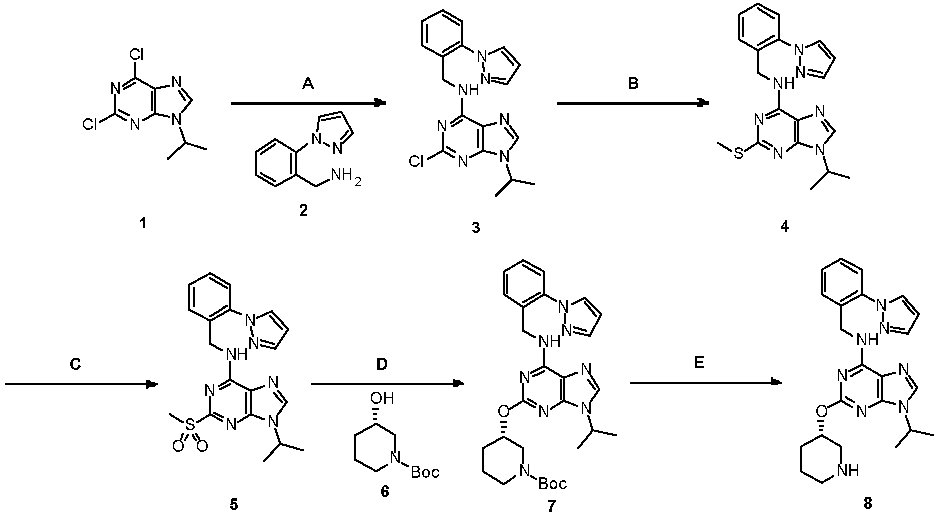

**Step A:** A mixture of 2,6-dichloro-9-isopropyl-9H-purine (1) (1.216 g, 1.0 eq), 1-(2-((chloro-15-azaneyl)methyl)phenyl)-1H-pyrazole (2) (1.434 g 1.3 eq) and N,N-Diisopropylethylamine (1.0 ml 1.5 eq) in isopropanol (20 mL) was heated at 80 °C for 3 h. The reaction was allowed to cool to room temperature, was diluted with ethyl acetate, washed with water and brine and dried over MgSO₄. Volatiles were removed under reduced pressure to provide compound 3 as a light yellowish solid (1.695 g, 88%). LCMS purity >95%.

**Step B:** A mixture of N-(2-(1H-pyrazol-1-yl)benzyl)-2-chloro-9-isopropyl-9H-purin-6-amine (3) (0.3 g, 1.0 eq) and sodium methanethiolate (0.229 g, 4 eq) in DMF (4 mL) was heated at 100 °C for 2 h. The reaction was allowed to cool to room temperature, was diluted with ethyl acetate, washed with water, sat. aq NaHCO₃ and brine and dried over MgSO₄. Volatiles were removed under reduced pressure to provide compound **4** as a light-yellowish solid (296 mg, quant.). LCMS purity >95%.

**Step C:** To a solution of N-(2-(1H-pyrazol-1-yl)benzyl)-9-isopropyl-2-(methylthio)-9H-purin-6-amine **(4)** (0.296 g, 1 eq) in DCM (15 mL) at 0 °C was added *m*CPBA (0.40 g, 2.2 eq). The mixture was stirred for 30 min while being allowed to warm to ambient temperature. The reaction was diluted with DCM, washed with sat. aq NaHCO₃ and brine and dried over MgSO₄. Volatiles were removed under reduced pressure to provide compound **5** as a yellowish solid (325 mg, quant.). LCMS purity >95%

**Step D:** A solution of N-(2-(1H-pyrazol-1-yl)benzyl)-9-isopropyl-2-(methylsulfonyl)-9H-purin-6-amine **(5)** (0.118 g, 1 eq), , tert-butyl (S)-3-hydroxypiperidine-1-carboxylate (6) (0.260 g 4.5 eq) and KOtBu (145 mg, 4.5 eq) in DMF (3 mL) was heated at 100 °C for 2 h. The reaction was allowed to cool to room temperature, was diluted with ethyl acetate, washed with water and brine and dried over MgSO₄. Purification by flash chromatography (silica, DCM to 5% MeOH) provided compound 7 as a colorless oily solid (90 mg, 59%). LCMS purity >95%

**Step E:** To a solution oftert-butyl (S)-3-((6-((2-(1H-pyrazol-1-yl)benzyl)amino)-9-isopropyl-9H-purin-2-yl)oxy)piperidine-1-carboxylate (7) (0.09 g, 1 eq) in dioxane (2 mL) was added HCl in dioxane (4M, 0.4 mL), which caused an oily precipitate. The reaction was diluted with MeOH (2 mL) and stirred at ambient temperature for 20 min. The mixture was dried under reduced pressure and the residue was purified by flash chromatography (C18, H₂O/ACN, 10% to 100%) to provide compound **8 (C002-S)** (3373-344) as a colorless solid (20 mg, 27%). ¹H NMR (400 MHz, DMSO) δ 9.65 (s, 1H), 8.87 (s, 1H), 8.31 (s, 1H), 8.22 (s, 1H), 8.13 (s, 1H), 7.81 (d, J = 1.1 Hz, 1H), 7.51 - 7.33 (m, 4H), 6.56 (t, J = 1.9 Hz, 1H), 5.08 (s, 1H), 3.44 (s, 2H), 3.22 (s, 1H), 3.12 - 2.95 (m, 3H), 1.84 (s, 2H), 1.7 (s, 2H), 1.56 - 1.4 (m, 6H).

### Further synthesis schemes:

Synthesis of TBM-C050 was carried out following the scheme given below:

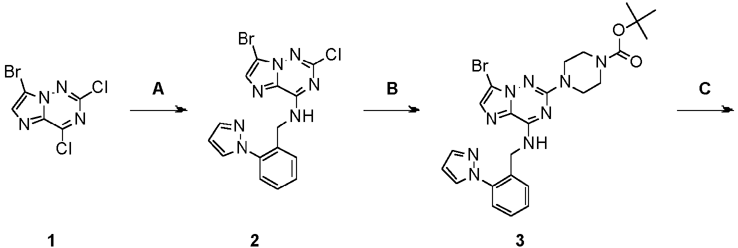

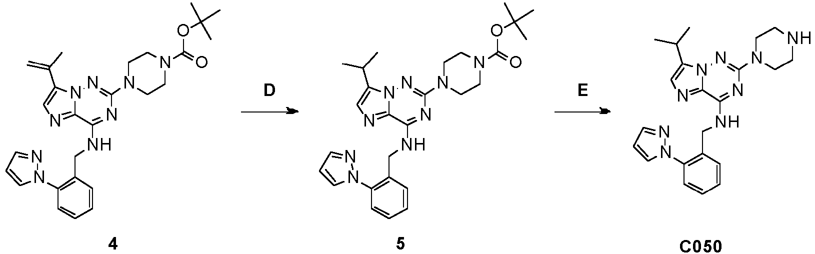

### Step A:

To a solution of amine HCl (0.26 g, 1.2 mmol) and DIPEA (0.35 g, 2.7 mmol) in DCM (30 mL) was added solution of compound **1** (0.33 g, 1.2 mmol) in DCM (3 mL) at -10°C. The reaction mixture was allowed to warm up to RT and stirred for 12 h. Then the mixture was diluted with water. The organic layer was separated, washed with water, brine, dried over Na₂SO₄ and evaporated in vacuo to give compound **2** (0.47 g, 94% yield).

### Step B:

A mixture of compound **2** (0.47 g, 1.17 mmol), amine (0.33 g, 1.76 mmol) and DIPEA (0.23 g, 1.76 mmol) in DMA (5 mL) was stirred in Ar at 130°C for 36 h. After the reaction completed, the mixture was cooled down to RT, diluted with water, extracted with EtOAc. The organic layer was washed with water, brine, dried and evaporated in vacuo to obtain compound 3 (0.59 g, 90% yield).

### Step C:

A degassed mixture of compound **3** (0.59 g, 1.07 mmol), isopropenylBPin (0.45 g, 2.67 mmol), Na₂CO₃ (0.34 g, 3.2 mmol) and BrettPhos PdG3 (97 mg, 0.1 mmol) in a mixture of Diox/H₂O 10/1 mL was stirred at 90°C for 16 h. After the reaction completed, the reaction mixture was cooled to RT, diluted with water, extracted with EtOAc. The organic layer was washed with water, brine, dried and evaporated in vacuo. The crude material was purified by prep-HPLC to afford compound **4** (0.33 g, 60% yield).

### Step D:

A suspension of compound **4** (0.33 g, 0.65 mmol) and Pd/C (70 mg, 10%, 0.065 mmol) in MeOH (20 mL) was hydrogenated at RT at 1 atm for 20 h. After the reaction completed, the catalyst was removed by filtration, the filtrate was evaporated to dryness to afford compound **5** (0.3 g, 91% yield).

### Step E:

Compound **5** (0.3 g, 5.8 mmol) was dissolved in DCM (10 mL) and 5 mL of HCl in ether was added. The reaction mixture was stirred at RT overnight, then evaporated to dryness. The residue was purified by prep-HPLC to afford the target compound **C050** (215 mg, 84% yield).

MS Calcd.: 417.52 MS Found: 418.0 [M + H]⁺.

¹H NMR (500 MHz, dmso) δ 8.73 (s, 1H), 8.12 (s, 1H), 7.78 (s, 1H), 7.50 (s, 1H), 7.38 (s, 3H), 7.13 (s, 1H), 6.54 (s, 1H), 4.59 (d, *J* = 6.1 Hz, 2H), 3.14 (dt, *J* = 13.7, 7.4 Hz, 1H), 2.64 (s, 4H), 2.55 (s, 4H), 1.26 (d, *J* = 6.9 Hz, 6H).

### Further synthesis schemes:

Synthesis of TBM-C013 was carried out following the scheme given below:

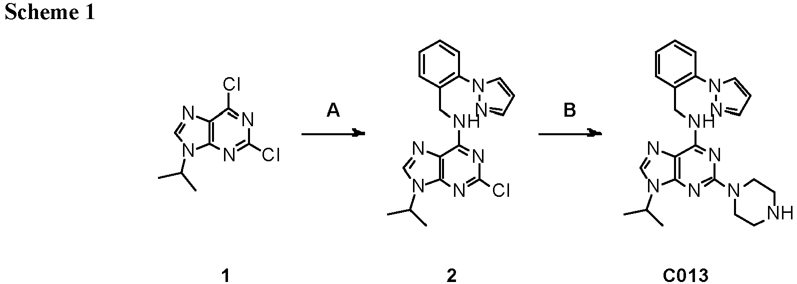

### Step A:

To a solution of [2-(1H-pyrazol-1-yl)phenyl]methanamine hydrochloride 5.99 g (28.55 mmol) in butanol (100 mL) TEA (6.88 g, 67.96 mmol) and compound **1** (6.28 g, 27.19 mmol) were added, then the obtained mixture was stirred overnight at 90°C. The reaction mixture is evaporated, then poured with water and placed in a cold ultrasonic bath for 30 minutes. The formed precipitate was filtered, washed with water, IPS, hexane, and dried under vacuum to obtained compound 2 (8.1 g, 81% yield).

### Step B:

A mixture of compound **2** (2.84 g, 7.7 mmol) and tert-butyl piperazine-1-carboxylate (1.58 g, 8.5 mmol) was dissolved in DMA (30 mL), then to obtained solution DIPEA (1.5 g, 11.59 mmol) was added. Then the obtained mixture was stirred overnight at 140°C. The reaction mixture was cooled to RT, poured with water and extracted with EtOAc. The organic layer was washed with water, dried over Na₂SO₄ and evaporated.

The obtained Boc-product was dissolved in 100 mL of 20% TFA in DCM and left to stir at RT for 3 hours, then the reaction mixture was evaporated, poured with 10% KOH and extracted with DCM. The organic layer is washed with water, dried over Na₂SO₄, and evaporated. The obtained crude material was purified by prep-HPLC to obtained target compound **C013** (1.1 g, 34% yeild, 95.17% purity).

MS Calcd.: 417.52 MS Found: 418.2 [M + H]⁺.

¹H NMR (500 MHz, dmso) δ 7.25 (t, *J =* 2.7 Hz, 1H), 7.16 (d, *J =* 2.7 Hz, 1H), 7.04 - 6.99 (m, 1H), 6.96 (d, *J* = 3.9 Hz, 1H), 6.32 (q, *J* = 3.3 Hz, 2H), 5.92 (q, *J* = 2.7 Hz, 1H), 5.87 (t, *J* = 5.9 Hz, 1H), 4.13 - 4.04 (m, 2H), 3.32 (t, *J* = 5.0 Hz, 4H), 3.26 (s, 3H), 2.46 (d, *J* = 9.9 Hz, 4H), 0.97 (s, 6H).

### Further synthesis schemes:

Synthesis of TBM-101 was carried out following the scheme given below:

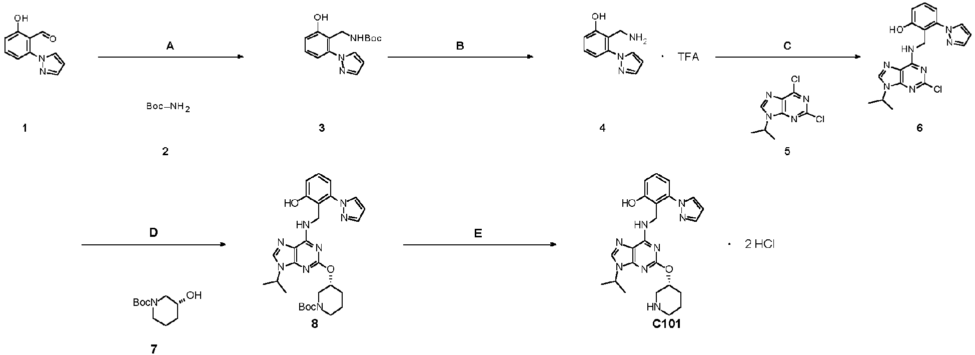

**Step A:** The reaction was carried out under an atmosphere of N₂. **1** (1.00 g, 5.31 mmol) and **2** (3.74 g, 31.9 mmol) were dissolved in MeCN (39 mL) and DCM (12 mL). Triethylsilane (5.1 mL, 31.9 mmol) and TFA (1.6 mL, 21.3 mmol) were successively added at room temperature, and the reaction mixture was stirred for 24 h before saturated aqueous NaHCO₃ solution was added. The phases were separated, and the aqueous layer was extracted with DCM (3x). The combined organic layers were washed with brine and dried over Na₂SO₄. The solvent was removed and the residue was purified by flash column chromatography (silica, cyclohexane → cyclohexane:EtOAc 93%) providing 3 (0.71 g) as a colorless solid. LC/MS purity >90%

**Step B: 3** (300 mg, 1.04 mmol) was dissolved in DCM (6.0 mL) and TFA (6.0 mL). The reaction was mixture was stirred for 4 h at room temperature before the solvent was removed. The residue was taken up in DCM and the solvent was removed (3 iterations) providing **4** (314 mg) as a colorless solid. LC/MS purity >90%

**Step C: 4** (314 mg, 1.04 mmol) and **5** (200 mg, 0.87 mmol) were dissolved in isopropanol (15.0 mL) DIPEA (0.60 mL, 3.46 mmol) was added and the reaction mixture was warmed to 80 °C. The reaction mixture was stirred at this temperature for 15 h before it was cooled to room temperature and the solvent was removed. The residue was purified by flash column chromatography (silica, cyclohexane → cyclohexane:EtOAc 60%) providing **6** (316 mg) as a colorless solid. LC/MS purity >90%

**Step D:** The reaction was carried out under an atmosphere of N₂. Sodium hydride (124 mg, 3.11 mmol) was suspended in NMP 10.0). **7** (548 mg, 2.72 mmol) was added and the reaction mixture was stirred for 1 h. **6** (300 mg, 0.78 mmol) was added and the reaction mixture was warmed to 100 °C. The reaction mixture was stirred at this temperature for 15 h before it was cooled to room temperature and saturated aqueous solution of NH₄Cl and EtOAc were added. The phases were separated, and the aqueous layer was extracted with EtOAc (3x). The combined organic layers were washed with brine and dried over Na₂SO₄. The solvent was removed and the residue was purified by flash column chromatography (silica, cyclohexane → cyclohexane: EtOAc 60%) followed by (C18, H₂O:MeCN 10% → MeCN) providing **8** (100 mg) as a colorless solid. LC/MS purity >90%

**Step E:** HCl (4 mol/L in dioxane, 0.20 mL) was added to a solution of **8** (60.0 mg, 0.11 mmol) in dioxane (1.0 mL) at room temperature. The reaction mixture was stirred for 2 h before additional HCl (4 mol/L in dioxane, 0.05 mL) was added. The reaction mixture was stirred for 2 h before it was diluted with MeOH, and the solvent was removed. The residue was taken up in MeOH and the solvent was removed (4 iterations) providing **C101** (35.0 mg) as a colorless solid. ¹H NMR (400 MHz, (CD₃)₂SO) δ 9.34 (bs, 1H), 8.83 (bs, 2H), 8.11 (d, *J* = 2.2 Hz, 1H), 7.98 (bs, 1H), 7.73 - 7.71 (m, 1H), 7.31 - 7.27 (m, 1H), 7.05 (d, *J* = 8.2 Hz, 1H), 6.89 - 6.87 (m, 1H), 6.48 - 6.47 (m, 1H), 5.24 - 5.18 (m, 1H), 4.78 - 4.66 (m, 1H), 4.56 - 4.44 (m, 2H), 3.42 - 3.34 (m, 1H), 3.27 - 3.19 (m, 1H), 3.09 - 3.02 (m, 2H), 2.04 - 1.83 (m, 3H), 1.78 - 1.70 (m, 1H), 1.53 (d, *J* = 6.6 Hz, 3H) 1.52 (d, *J* = 6.6 Hz, 3H) ppm.

### Further synthesis schemes:

Synthesis of TBM-C025 was carried out following the scheme given below:

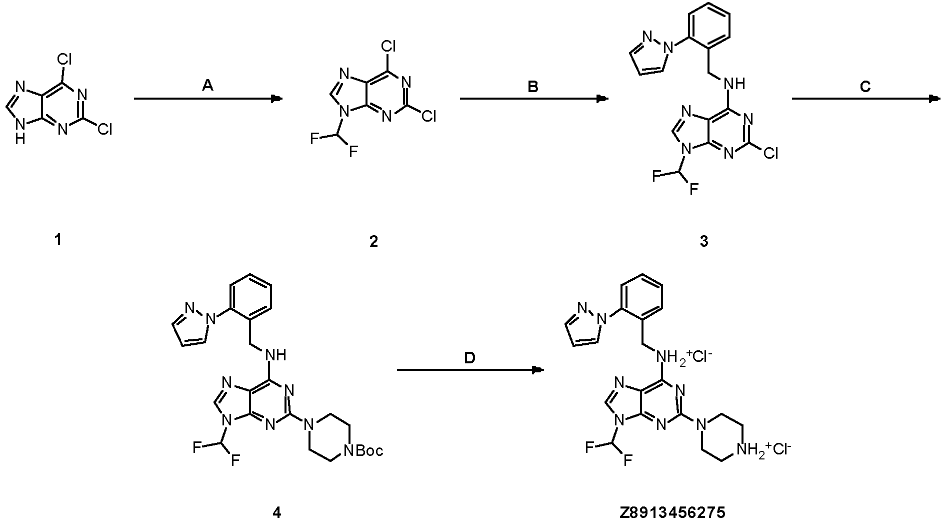

**Step A:** 2,6-dichloropurine (1.00 g, 52.9 mmol) was dissolved in DMF (10 ml) and ethyl 2-bromo-2,2-difluoroacetate (2.15 g, 10.6 mmol) and K₃PO₄ (2.81 g, 13.2 mmol) were added at room temperature. The reaction was stirred at room temperature for 18 h. H₂O was added, the mixture was extracted with EtOAc. The organic layer was further washed with H₂O (2x) brine, dried (Na₂SO₄) and evaporated *in vacuo.* The residue was purified by FC (10 g SiO₂, CyH/EtOAc 100:0 - 8:2 - 1:1), which afforded compound **2** (170 mg, 0.711 mmol, 13%) as a colorless solid.

**Step B: 2** (152 mg, 0.636 mmol), [2-(1H-pyrazol-1-yl)phenyl]methanamine hydrochloride (173 mg, 0.825 mmol) were dissolved in iPrOH (4 mL) and DIPEA (0.28 mL, 1.59 mmol) was added at rt. The reaction mixture was heated to 80 °C for 4h and subsequently evaporated *in vacuo.* Purification by FC (12 g SiO₂, CyH/EtOAc 100:0 - 1:1). Compound **3** (191 mg, 0.508 mmol, 80%) was obtained as a colorless solid.

**Step C: 3** (97.0 mg, 0.258 mmol) and Boc-piperazine (72 mg, 0.39 mmol) were dissolved in DMSO (2.6 mL), DIPEA (0.57 mL, 0.41 mmol) was added at rt and the mixture was heated to 140 °C for 18 h. Then, the mixture was diluted with EtOAc, washed with H₂O (2x), 1M KHSO₄ and brine. The organic phase was dried (Na₂SO₄) and evaporated in vacuo. Purification by FC (5 g SiO₂, CyH/EtOAc 100:0 - 6:4 - 4:6) afforded compound **4** (37.9 mg, 0.0721 mmol) as a colorless amorphous solid.

**Step D: 4** (31.0 mg, 59.0 µmol) was dissolved in DCM (0.2 mL) and 4M HCl in dioxane (0.29 mL) was added at rt. After 20 h, the mixture was evaporated *in vacuo.* Lyophilization from H₂O afforded the title compound **Z8913456275 (C025-CHF₂)** (26.2 mg, 52.6 µmol, 89%) as an off-white solid.

¹H NMR (400 MHz,DMSO-d₆) δ 9.31 (s, 2H), 8.38 (s, 1H), 8.24 (s, 1H), 8.16 (d, *J* = 2.3 Hz, 1H), 8.02 - 7.63 (m, 2H), 7.55 - 7.47 (m, 1H), 7.45 - 7.32 (m, 3H), 6.56 (t, *J* = 1.9 Hz, 1H), 4.58 (d, *J =* 4.9 Hz, 2H), 3.75 (s, 4H), 3.01 (s, 4H).

### Further synthesis schemes:

Synthesis of TBM-C023 was carried out following the scheme given below:

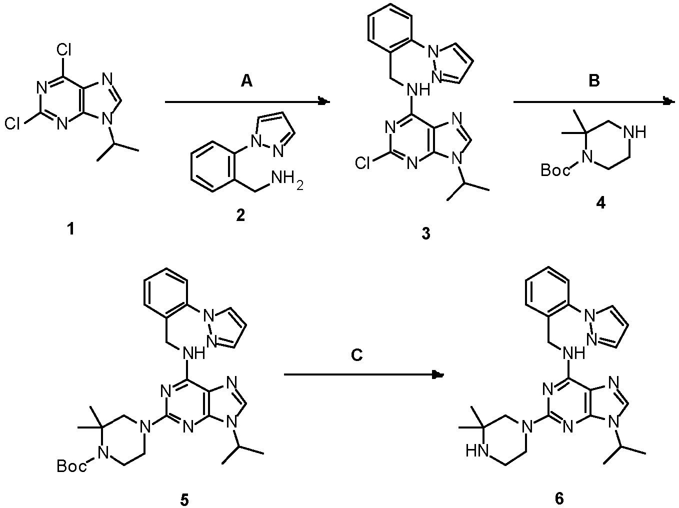

**Step A:** A mixture of 2,6-dichloro-9-isopropyl-9H-purine **(1)** (1.216 g, 1.0 eq), 1-(2-((chloro-l5-azaneyl)methyl)phenyl)-1H-pyrazole (2) (1.434 g 1.3 eq) and N,N-Diisopropylethylamine (1.0 ml 1.5 eq) in isopropanol (20 mL) was heated at 80 °C for 3 h. The reaction was allowed to cool to room temperature, was diluted with ethyl acetate, washed with water and brine and dried over MgSO₄. Volatiles were removed under reduced pressure to provide compound 3 as a light yellowish solid (1.695 g, 88%). LCMS purity >95%.

**Step B:** A mixture of N-(2-(1H-pyrazol-1-yl)benzyl)-2-chloro-9-isopropyl-9H-purin-6-amine **(3)** (0.4 g, 1.0 eq), tert-butyl 2,2-dimethylpiperazine-1-carboxylate **(4)** (0.582 g, 2.5 eq) and N,N-Diisopropylethylamine (0.28 ml, 2.0 eq) in DMSO (10 mL) was heated at 140 °C for 9 h and another 4 h at 150 °C. The reaction was allowed to cool to room temperature, was diluted with ethyl acetate, washed with water and brine and dried over MgSO₄. Volatiles were removed under reduced pressure and the crude product was purified by flash chromatography (silica, CH with 5% to 80% EtOAc) to provide compound 5 as a colorless oil, that still contained ca 30% of unreacted compound **3** (366 mg).

**Step C:** To a solution of tert-butyl 4-(6-((2-(1H-pyrazol-1-yl)benzyl)amino)-9-isopropyl-9H-purin-2-yl)-2,2-dimethylpiperazine-1-carboxylate **(5)** (0.366g) in dioxane (6 mL) was added HCl in dioxane (4 M, 1.8 mL), which caused an oily precipitate. The reaction was diluted with methanol (6 mL), stirred at room temperature for 2.5 h and volatiles were removed under reduced pressure. The crude product was purified by flash chromatography (C18, H₂O/ACN, 10% to 100%) to provide compound **6 (C023b)** (3363-952) as a colorless solid (118 mg, 39%). ¹H NMR (400 MHz, DMSO) δ 9.35 (s, 2H), 8.2 (s, 1H), 8.14 (d, J = 2.3 Hz, 1H), 8.05 (s, 1H), 7.8 (d, J = 1.4 Hz, 1H), 7.5 - 7.45 (m, 1H), 7.44 - 7.4 (m, 1H), 7.39 - 7.33 (m, 2H), 6.56 (t, J = 2.1 Hz, 1H), 4.65 - 4.49 (m, 3H), 3.8 (s, 2H), 3.58 (s, 2H), 3.02 (s, 2H), 2.07 (s, 0H), 1.47 (d, J = 6.8 Hz, 6H), 1.15 (s, 6H)

### Further synthesis schemes:

Synthesis of TBM-C008 was carried out following the scheme given below:

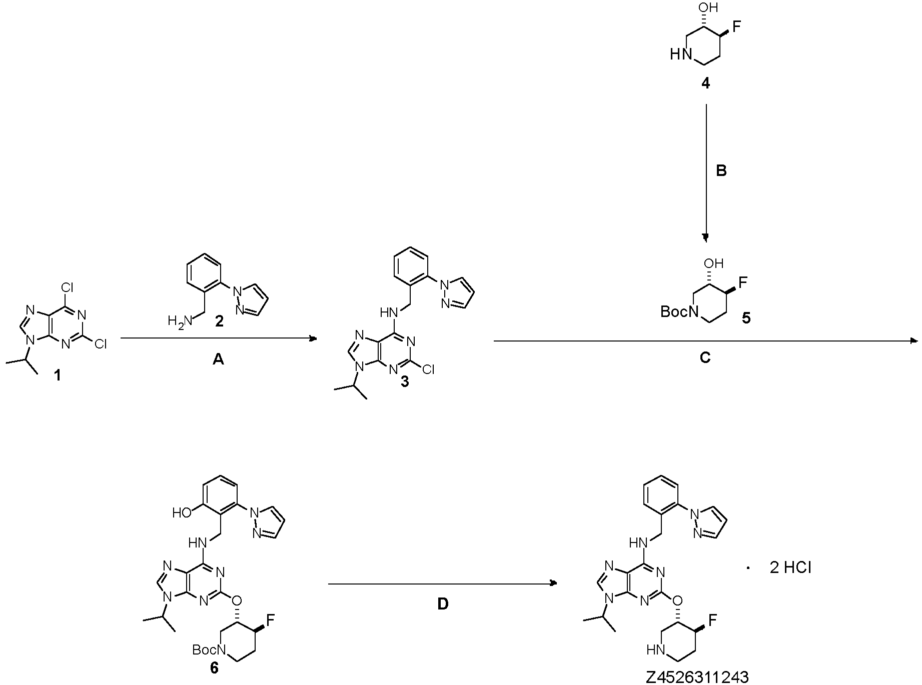

**Step A:** Diisopropylethylamine (9.0 mL, 51.9 mmol) was added to a suspension of **1** (3.00 g, 13.0 mmol) and 2 (hydrochloride salt, 3.27 g, 15.6 mmol) in isopropanol (150 mL) the reaction mixture was heated to 80 °C and stirred for 15.5 h. The solvent was evaporated and the residue was purified by flash column chromatography (silica, cyclohexane → cyclohexane:EtOAc 34% to cyclohexane:EtOAc 66%) providing 3 (4.47 g) as a colorless solid.

**Step B:** The reaction was carried out under an atmosphere of N₂. Di-tert-butyl dicarbonate (4.62 g, 21.2 mmol) was dissolved in DCM (36 mL) and added to a solution of **4** (hydrochloride salt, 3.00 g, 19.3 mmol) and triethylamine (6.7 mL, 48.2 mmol) in DCM (60 mL) at room temperature. The reaction mixture was stirred for 15 h and then poured into a separatory funnel filled with water (80 mL). The phases were separated, and the aqueous phase was extracted with DCM (3x 80 mL). The combined organic phases were washed with brine (150 mL), dried over Na₂SO₄ and the solid was filtered off (DCM rinse). The solvent was evaporated and the residue was purified by flash column chromatography (silica, cyclohexane to cyclohexane:EtOAc 66%) providing **5** (3.63 g) as a colorless solid.

**Step C:** The reaction was carried out under an atmosphere of N₂. Anhydrous dioxane (40 mL) was added to a 250 mL round bottom flask filled with **3** (1.51 g, 4.11 mmol), **5** (2.70 g, 12.3 mmol), Cs₂CO₃ (4.01 g, 12.3 mmol) and Xantphos (0.48 mg, 0.82 mmol). The reaction mixture was sparged with N₂ for 15 minutes. Tris(dibenzylideneacetone)dipalladium(0) (0.75 g, 0.82 mmol) was added and the reaction mixture was sparged with N₂ for 45 minutes before it was heated to 120 °C and stirred under an N₂ atmosphere. The reaction mixture was stirred for 18 h at 120 °C and then cooled to room temperature. The solids were filtered off over Celite (EtOAc rinse) and the solvent was removed. The residue was purified by flash column chromatography (first: silica, cyclohexane to EtOAc; second: C18-silica, water:MeCN 90:10 to MeCN) providing 6 (0.45 g) as a colorless solid.

**Step D:** The reaction was carried out in two 700 mg batches. HCl (4 mol/L in dioxane, 5.0 mL) was added to a solution of **6** (700 mg, 1.28 mmol) in dioxane (10.0 mL) at room temperature. The reaction mixture was stirred for 19.5 h. The solvent was removed, and the residue was taken up in MeOH (3.0 mL), which was then removed again. This process was performed for four timed and the obtained residues were combined and purified by flash column chromatography (C18-silica, water:MeCN 10:90 to MeCN) providing **Z4526311243** (**C008**) (950 mg) as a colorless solid. ¹H NMR (400 MHz, (CD₃)₂SO) δ 9.25 (bs, 2H), 8.32 (s, 1H), 8.21 (s, 1H), 8.12 (s, 1H), 7.82 (s, 1H), 7.46 - 7.36 (m, 4H), 6.56 (s, 1H), 5.25 (s, 1H), 4.93 (d, *J* = 48.6 Hz, 1H), 4.68 - 4.56 (m, 3H), 3.36 - 3.00 (m, 4H), 2.30 - 2.12 (m, 1H), 2.10 - 1.96 (m, 1H), 1.50 (d, *J* = 6.0 Hz, 6H) ppm.

### Further synthesis schemes:

Synthesis scheme for stereospecific piperidine derivatives:

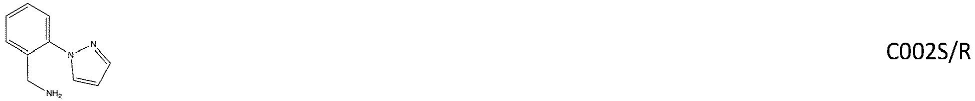

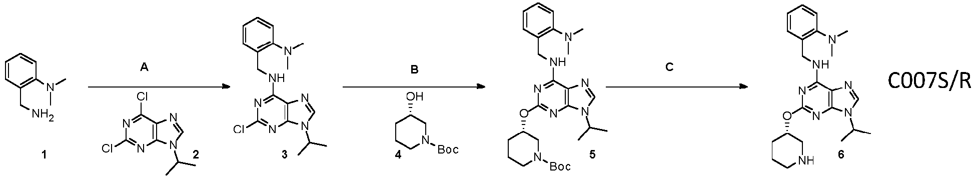

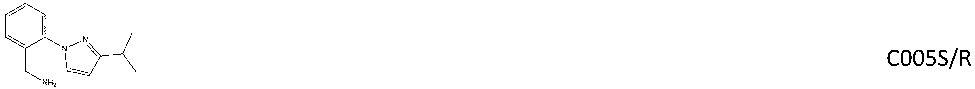

IC₅₀ values of separated enantiomers C008 and C019 were determined - measured at the KM of kinases CDK7/CycH/MAT1 versus (next related) CDK2/cycE1:

| | **CDK2/CycE1, 1st** | **CDK2/CycE1, 2nd** | | | **CDK7/CycH/MAT1, 1st** | **CDK7/CycH/MAT1, 2nd** |
|---|---|---|---|---|---|---|
| Compound ID | IC₅₀ (M) | IC₅₀ (M) | | Compound ID | IC₅₀ (M) | IC₅₀ (M) |
| CDK2-C008_1 | 2.15 10⁻⁶ | 2.08 10⁻⁶ | | CDK7-C008_1 | 2.25 10⁻⁷ | 1.97 10⁻⁷ |
| CDK2-C008_2 | 3.78 10⁻⁸ | 3.90 10⁻⁸ | | CDK7-C008_2 | 6.61 10⁻⁹ | 7.42 10⁻⁹ |
| CDK2-C019_1 | 1.05 10⁻⁵ | 1.11 10⁻⁵ | | CDK7-C019_1 | 1.78 10⁻⁸ | 1.98 10⁻⁸ |
| CDK2-C019_2 | 1.21 10⁻⁵ | 1.07 10⁻⁵ | | CDK7-C019_2 | 6.60 10⁻⁸ | 8.62 10⁻⁸ |
| CDK2-C020 | 1.12 10⁻⁶ | 1.01 10⁻⁶ | | CDK7-C020 | 1.69 10⁻⁸ | 1.20 10⁻⁸ |
| CDK2-C023 | 9.39 10⁻⁷ | 7.28 10⁻⁷ | | CDK7-C023 | 4.00 10⁻⁸ | 4.37 10⁻⁸ |
| Staurosporine (RBE) | 1.24 10⁻⁸ | 1.16 10⁻⁸ | | Staurosporine (RBE) | 1.74 10⁻⁷ | 1.67 10⁻⁷ |

### Selection of IC₅₀ values:

| | **CDK2/CycE1** | **CDK7/CycH/MAT1** | **CDK9/CycT1** |
|---|---|---|---|
| | IC₅₀ (M) | IC₅₀ (M) | IC₅₀ (M) |
| C001 | 1.89 10⁻⁷ | 2.29 10⁻⁸ | 1.79 10⁻⁵ |
| C001 | 2.48 10⁻⁷ | 1.72 10⁻⁸ | 1.36 10⁻⁵ |
| C001 | 4.32 10⁻⁷ | 1.56 10⁻⁸ | 1.19 10⁻⁵ |
| C002 | 1.41 10⁻⁸ | 3.90 10⁻⁹ | 1.54 10⁻⁶ |
| C002 | 1.57 10⁻⁸ | 3.29 10⁻⁹ | 2.12 10⁻⁶ |
| C002 | 1.24 10⁻⁸ | 2.61 10⁻⁹ | 1.88 10⁻⁶ |
| LDC4297 | 1.67 10⁻⁸ | 5.29 10⁻⁹ | 1.92 10⁻⁶ |
| LDC4297 | 1.52 10⁻⁸ | 4.68 10⁻⁹ | 1.75 10⁻⁶ |
| LDC4297 | 1.68 10⁻⁸ | 5.89 10⁻⁹ | 1.63 10⁻⁶ |
| C013 | 5.42 10⁻⁷ | 5.99 10⁻⁹ | 1.93 10⁻⁵ |
| C013 | 5.02 10⁻⁷ | 4.72 10⁻⁹ | 1.62 10⁻⁵ |
| C032 | 4.15 10⁻⁶ | 1.80 10⁻⁷ | >2 10⁻⁴ |
| C032 | 4.28 10⁻⁶ | 2.06 10⁻⁷ | >2 10⁻⁴ |
| C035 | 4.78 10⁻⁷ | 2.63 10⁻⁸ | 2.03 10⁻⁵ |
| C035 | 4.81 10⁻⁷ | 3.03 10⁻⁸ | 2.48 10⁻⁵ |
| C003 | 1.67 10⁻⁸ | 8.13 10⁻⁹ | 1.28 10⁻⁷ |
| C003 | 1.18 10⁻⁸ | 1.64 10⁻⁸ | 9.72 10⁻⁸ |
| | | | |
| C033 | 3.28 10⁻⁷ | 4.18 10⁻⁸ | >5 10⁻⁵ |
| C033 | 3.88 10⁻⁷ | 3.58 10⁻⁸ | >5 10⁻⁵ |
| C034 | 2.71 10⁻⁵ | 2.00 10⁻⁷ | >2 10⁻⁴ |
| C034 | 5.61 10⁻⁵ | 2.28 10⁻⁷ | >2 10⁻⁴ |
| C034 | 9.96 10⁻⁵ | 1.90 10⁻⁷ | >2 10⁻⁴ |
| LDC4297 | 1.81 10⁻⁸ | 5.02 10⁻⁹ | 2.23 10⁻⁶ |
| LDC4297 | 2.03 10⁻⁸ | 5.03 10⁻⁹ | 2.18 10⁻⁶ |

| | **IC50 CDK7-1** | **IC50 CDK7-2** | **average** | **IC₅₀ CDK2/cycE1-1___-2** | | **affinity** | **selectivity** | **average** | **STDV** |
|---|---|---|---|---|---|---|---|---|---|
| C027 | 1.58 10⁻⁸ | 1.70 10⁻⁸ | 1.64 10⁻⁸ | 7.64 10⁻⁷ | 1.39 10⁻⁶ | 4.83 10⁺¹ | 8.14 10⁺¹ | 6.49 10⁺¹ | 23.4419078 |
| C007-S | 4.03 10⁻⁷ | 4.38 10⁻⁷ | 4.21 10⁻⁷ | 4.33 10⁻⁶ | 7.46 10⁻⁶ | 1.07 10⁺¹ | 1.70 10⁺¹ | 1.39 10⁺¹ | 4.44911717 |
| C012 | 3.35 10⁻⁸ | 3.67 10⁻⁸ | 3.51 10⁻⁸ | 6.95 10⁻⁶ | >1 10⁻⁵ | 2.08 10⁺² | 2.73 10⁺² | 2.40 10⁺² | 45.9019291 |
| C008 | 1.49 10⁻⁸ | 8.87 10⁻⁹ | 1.19 10⁻⁸ | 5.45 10⁻⁸ | 7.03 10⁻⁸ | 3.65E+0 0 | 7.92E+00 | 5.79E+0 0 | 3.01893439 |
| C026 | 1.62 10⁻⁸ | 1.72 10⁻⁸ | 1.67 10⁻⁸ | 1.57 10⁻⁶ | 2.00 10⁻⁶ | 9.67 10⁺¹ | 1.16 10⁺² | 1.06 10⁺² | 13.887606 |
| C014 | 9.18 10⁻⁹ | 9.15 10⁻⁹ | 9.17 10⁻⁹ | 8.95 10⁻⁷ | 1.28 10⁻⁶ | 9.75 10⁺¹ | 1.40 10⁺² | 1.19 10⁺² | 30.0315332 |
| C050 | 6.48 10⁻⁹ | 5.16 10⁻⁹ | 5.82 10⁻⁹ | 4.08 10⁻⁷ | 5.90 10⁻⁷ | 6.30 10⁺¹ | 1.14 10⁺² | 8.87 10⁺¹ | 36.3314033 |
| C054 | 4.78 10⁻⁹ | 5.91 10⁻⁹ | 5.35 10⁻⁹ | 5.33 10⁻⁷ | 5.97 10⁻⁷ | 1.11 10⁺² | 1.01 10⁺² | 1.06 10⁺² | 7.44186109 |
| C025 | 2.32 10⁻⁸ | 2.21 10⁻⁸ | 2.27 10⁻⁸ | 1.18 10⁻⁶ | 1.72 10⁻⁶ | 5.10 10⁺¹ | 7.78 10⁺¹ | 6.44 10⁺¹ | 18.9925199 |
| C017 | 7.30 10⁻⁸ | 7.01 10⁻⁸ | 7.16 10⁻⁸ | 4.20 10⁻⁶ | 6.48 10⁻⁶ | 5.75 10⁺¹ | 9.24 10⁺¹ | 7.50 10⁺¹ | 24.6621704 |
| C011 | 7.82 10⁻⁸ | 1.04 10⁻⁷ | 9.12 10⁻⁸ | 1.24 10⁻⁵ | 1.41 10⁻⁵ | 1.59 10⁺² | 1.36 10⁺² | 1.47 10⁺² | 16.1899855 |
| C013 | 1.19 10⁻⁸ | 1.37 10⁻⁸ | 1.28 10⁻⁸ | 1.61 10⁻⁶ | 1.86 10⁻⁶ | 1.36 10⁺² | 1.36 10⁺² | 1.36 10⁺² | 0.02959565 |
| C005-S | 5.22 10⁻⁸ | 1.05 10⁻⁷ | 7.85 10⁻⁸ | 8.37 10⁻⁶ | 1.06 10⁻⁵ | 1.60 10⁺² | 1.01 10⁺² | 1.31 10⁺² | 42.3391827 |
| C018 | 1.02 10⁻⁸ | 1.17 10⁻⁸ | 1.10 10⁻⁸ | 6.52 10⁻⁷ | 8.45 10⁻⁷ | 6.39 10⁺¹ | 7.22 10⁺¹ | 6.80 10⁺¹ | 5.86695988 |
| C021 | 1.20 10⁻⁸ | 1.59 10⁻⁸ | 1.40 10⁻⁸ | 2.40 10⁻⁶ | 3.96 10⁻⁶ | 2.00 10⁺² | 2.48 10⁺² | 2.24 10⁺² | 34.5170845 |
| Stauros porine | 1.62 10⁻⁷ | 2.13 10⁻⁷ | 1.88 10⁻⁷ | 6.55 10⁻⁹ | 1.10 10⁻⁸ | 4.04 10⁻² | 5.15 10⁻² | 4.60 10⁻² | 0.00783895 |

| | | | | | | | | | |
|---|---|---|---|---|---|---|---|---|---|
| Result: Piperazine group C013 and derivatives C012, C014, C019, C021 show generally higher specificity than members of the piperidine group (C002, C008, LDC4297). Comparative cmpd TBM-C003 shows limited specificity demonstrated by elevated affinity towards CDK2 and CDK9. | | | | | | | | | |

### IC₅₀ values of lead compound C002 versus compound C054 at physiological ATP m (1 mM):

| | **Compound IC₅₀* (M):** | |
|---|---|---|
| Compound ID: | CDK2/cyclin E | CDK7/cyclin H |
| TBM-C002-R | 3.34 10⁻⁸ | 5.14 10⁻⁹ |
| TBM-C002-R | 2.97 10⁻⁸ | 4.73 10⁻⁹ |
| TBM-C002-R | 3.29 10⁻⁸ | 5.15 10⁻⁹ |
| TBM-C054 | 4.11 10⁻⁶ | 5.38 10⁻⁹ |
| TBM-C054 | 4.06 10⁻⁶ | 5.18 10⁻⁹ |
| TBM-C054 | 3.73 10⁻⁶ | 6.07 10⁻⁹ |
| IC₅₀ (M) Control Cmpd | 2.34 10⁻⁸ | 9.72 10⁻⁷ |
| Control Cmpd ID | Staurosporine | Staurosporine |

| | | |
|---|---|---|
| Result: C054 displays approximately 2 orders of magnitude higher specificity for CDK7 than C002 (R form) at physiological ATP. | | |

### Further experimental data:

| **Compound Name** | **ID50**_**5d; Panc89 (uM)** |
|---|---|
| | |
| TBM-C001-R | 0,3 |
| TBM-C002-R | 0,02 |
| TBM-C002-S | 3,3 |
| TBM-C005-R | 0,02 |
| TBM-C005-S | 0,75 |
| TBM-C007-R | 0,09 |
| TBM-C007-S | 4,3 |
| TBM-C008-1 | 1,4 |
| TBM-C008-2 | 0,09 |
| TBM-C013 | 0,13 |
| TBM-C014 | 0,04 |
| TBM-C015 | 12,4 |
| TBM-C016 | 6 |
| TBM-C017 | 1,95 |
| TBM-C018 | 0,3 |
| TBM-C019_1 | 0,09 |
| TBM-C019_2 | 0,4 |
| TBM-C021 | 0,06 |
| TBM-C022 | 1,05 |
| TBM-C023 | 0,73 |
| TBM-C025 | 0,28 |
| TBM-C026 | 0,1 |
| TBM-C027 | 0,11 |
| TBM-C031 | 2,5 |
| TBM-C032 | 3,1 |
| TBM-C033 | 0,08 |
| TBM-C034 | 1,55 |
| TBM-C035 | 0,33 |
| TBM-C050 | 0,075 |
| TBM-C054 | 0,0065 |
| TBM-C055 | 0,0125 |
| TBM-C100 | 3,6 |
| TBM-C101 | 6 |
| TBM-C103 | >40 |

| | |
|---|---|
| ID50_5d Panc89: concentration that leads to 50% growth reduction of Panc89 after 5d; R,S stands for defined stereospecific forms of the respective compounds; Cxxx_1/Cxxx_2 for undefined enantiomeric forms of the respective compound. | |

## Claims

1. A compound according to general formula (I) 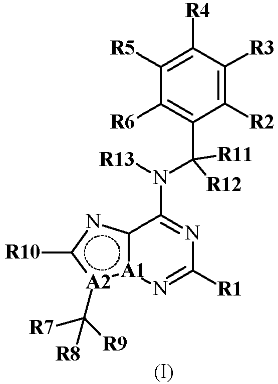 wherein
one of **A1** and **A2** means N and the other one of **A1** and A2 means C;
**R1** means -F, -Cl, -NHC₁₋₆-alkyl, -NHC₁₋₆-alkylene-OH, -NHC₁₋₆-alkylene-NH₂, -(hetero-)cycloalkyl or -O-(hetero-)cycloalkyl;
wherein -(hetero-)cycloalkyl or -O-(hetero-)cycloalkyl is optionally substituted with one, two or three substituents independently of one another selected from -F, -Cl, -CN, -C₁₋₆-alkyl, -C₁₋₆-alkylene-OH, -C₁₋₆-alkylene-CO₂H, -C₁₋₆-alkylene-C(=O)OC₁₋₆-alkyl, -C₁₋₆-alkylene-OC(=O)C₁₋₆-alkyl, -OH, -OC₁₋₆-alkyl, -OC₁₋₆-alkylene-OH, -O(CH₂CH₂O)ₙ-H with n = 1-50, -OC(=O)C₁₋₆-alkyl, and -(hetero-)aryl;
wherein -(hetero-)aryl is optionally substituted with one, two or three substituents independently of one another selected from -F, -Cl, -CN, -C₁₋₆-alkyl, -C₁₋₆-alkylene-OH, -C₁₋₆-alkylene-CO₂H, -C₁₋₆-alkylene-C(=O)OC₁₋₆-alkyl, -C₁₋₆-alkylene-OC(=O)C₁₋₆-alkyl, -OH, -OC₁₋₆-alkyl, -OC₁₋₆-alkylene-OH, -O(CH₂CH₂O)ₙ-H with n = 1-50, and -OC(=O)C₁₋₆-alkyl;
**R2** means -N(C₁₋₆-alkyl)₂ or -(hetero-)aryl;
wherein -(hetero-)aryl is optionally substituted with one, two or three substituents independently of one another selected from -F, -Cl, -CN, -C₁₋₆-alkyl, -C₁₋₆-alkylene-OH, - C₁₋₆-alkylene-CO₂H, -C₁₋₆-alkylene-C(=O)OC₁₋₆-alkyl, -C₁₋₆-alkylene-OC(=O)C₁₋₆-alkyl, - OH, -OC₁₋₆-alkyl, -OC₁₋₆-alkylene-OH, -O(CH₂CH₂O)ₙ-H with n = 1-50, and -OC(=O)C₁₋₆-alkyl;
**R3, R4, R5, R6, R7, R8, R9, R10, R11 and R12** independently of one another mean -H, -F, -Cl, -CN, -C₁₋₆-alkyl, -C₁₋₆-alkylene-OH, -C₁₋₆-alkylene-CO₂H, -C₁₋₆-alkylene-C(=O)OC₁₋₆-alkyl, -C₁₋₆-alkylene-OC(=O)C₁₋₆-alkyl, -OH, -OC₁₋₆-alkyl, -OC₁₋₆-alkylene-OH, -O(CH₂CH₂O)ₙ-H with n = 1-50, or -OC(=O)C₁₋₆-alkyl;
or **R2** and **R3** together with the carbon atoms to which they are attached form a ring and mean -CH=CH-O-;
and
**R13** means -H or -C₁₋₆-alkyl;
or a physiologically acceptable salt thereof.

2. The compound according to claim 1, wherein **R1** means -(hetero-)cycloalkyl or -O-(hetero-)cycloalkyl; preferably -O-piperidinyl or -piperazinyl; wherein the optional substituents are defined as in claim 1.

3. The compound according to claim 1 or 2, wherein **R2** means -N(CH₃)₂ or -pyrazolyl, wherein the optional substituents are as defined in claim 1 or claim 2.

4. The compound according to any of the preceding claims, which is of general formula (II-A) or (II-B) 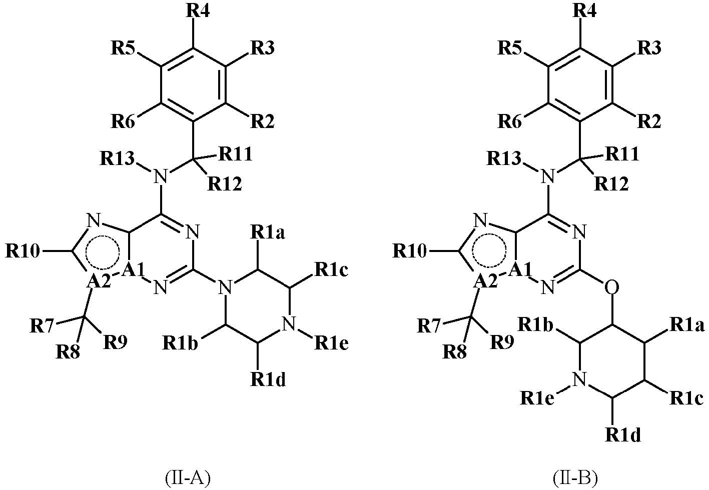 wherein
**R1a**, **R1b, R1c** and **R1d** independently of one another mean -H, -F, -Cl, -CN, -C₁₋₆-alkyl, -OH, -C₁₋₆-alkylene-OH, or -O-C₁₋₆-alkyl; and
**R1e** means -H or -C₁₋₆-alkyl.

5. The compound according to claim 4, wherein **R1b** and **R1d** mean -H.

6. The compound according to any of the preceding claims, which is of general formula (III) 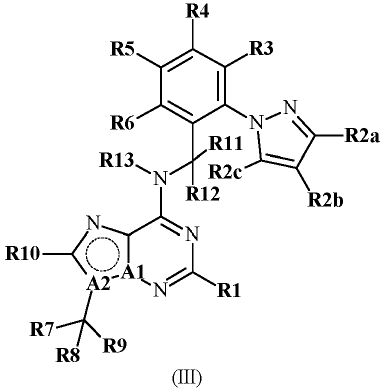 wherein
**R2a, R2b,** and **R2c** independently of one another mean -H, -F, -Cl, -CN, -C₁₋₆-alkyl, -OH, -C₁₋₆-alkylene-OH, or -O-C₁₋₆-alkyl.

7. The compound according to any of the preceding claims, which is of general formula (IV-A) or (IV-B) 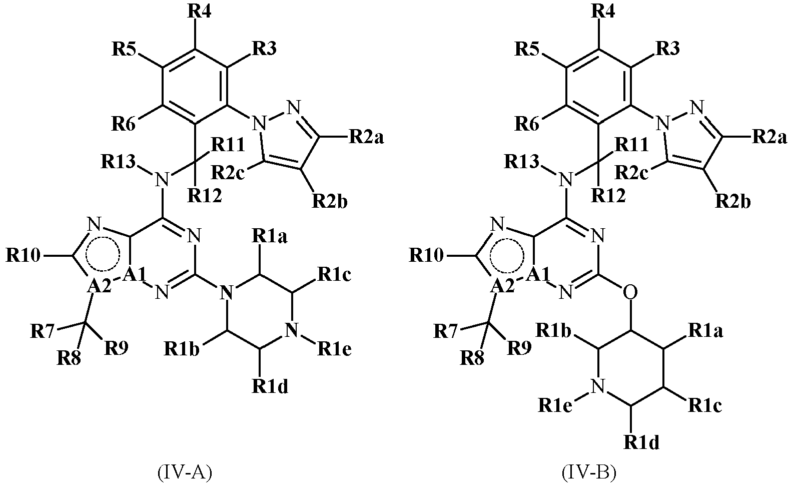 wherein
**R1a**, **R1b, R1c** and **R1d** independently of one another mean -H, -F, -Cl, -CN, -C₁₋₆-alkyl, -OH, -C₁₋₆-alkylene-OH, or -O-C₁₋₆-alkyl;
**R1e** means -H or -C₁₋₆-alkyl; and
**R2a, R2b,** and **R2c** independently of one another mean -H, -F, -Cl, -CN, -C₁₋₆-alkyl, -OH, -C₁₋₆-alkylene-OH, or -O-C₁₋₆-alkyl.

8. The compound according to any of the preceding claims, wherein **R3** means
- -H, -C₁₋₆-alkylene-OH, -OH, -OC₁₋₆-alkyl, or -O(CH₂CH₂O)ₙ-H with n = 1-50;
- preferably -H, -CH₂CH₂OH, -OH, -OC₁₋₆-alkyl or -O(CH₂CH₂O)ₙ-H with n = 1-20;
- more preferably -OC₁₋₆-alkyl; and
- even more preferably -OCH₃.

9. The compound according to any of the preceding claims, wherein
- **R4** and **R6** mean -H; and/or
- **R5** means -H or -OH.

10. The compound according to any of the preceding claims, wherein one of **R7**, **R8** and **R9** means
- H and the other two of **R7, R8** and **R9** both mean -CH₃, -F, or -CH₂OH.

11. The compound according to any of the preceding claims, wherein
- **R10,** R11 and/or **R12** mean -H; and/or
- **R13** means -H or -CH₃; preferably -H.

12. The compound according to any of the preceding claims, wherein
- n = 1-20; and/or
- -C₁₋₆-alkyl is -C₁₋₄-alkyl; and/or
- -C₁₋₆-alkylene is -C₁₋₄-alkylene.

13. The compound according to any of the preceding claims, which is selected from the group consisting of

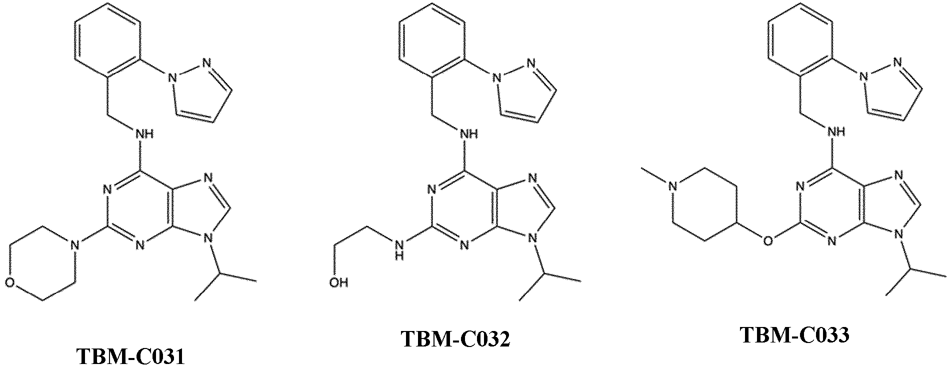

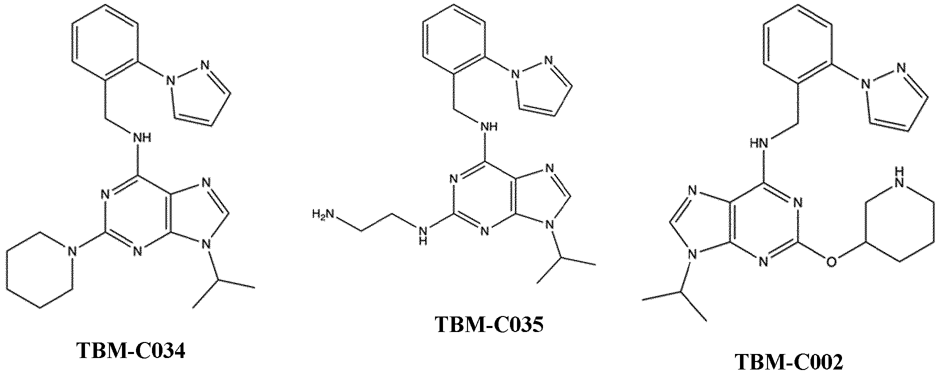

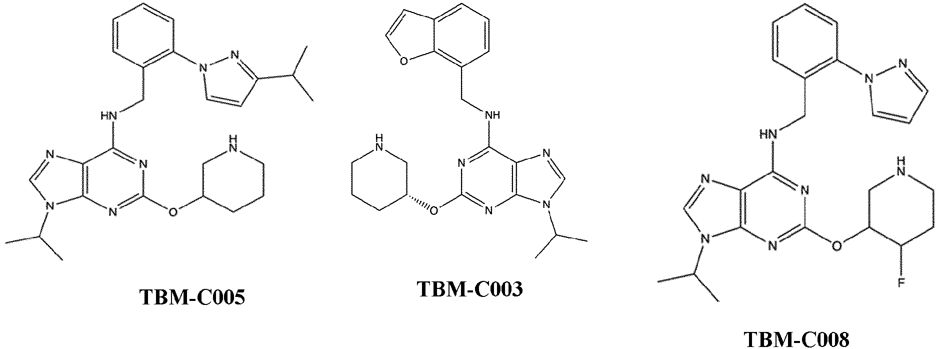

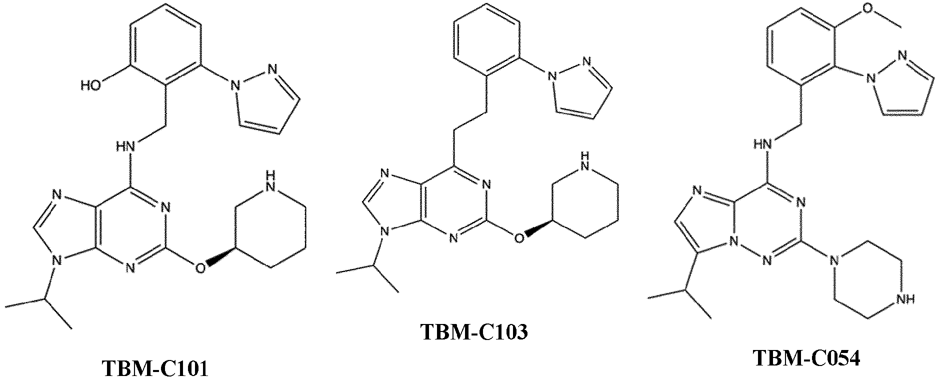

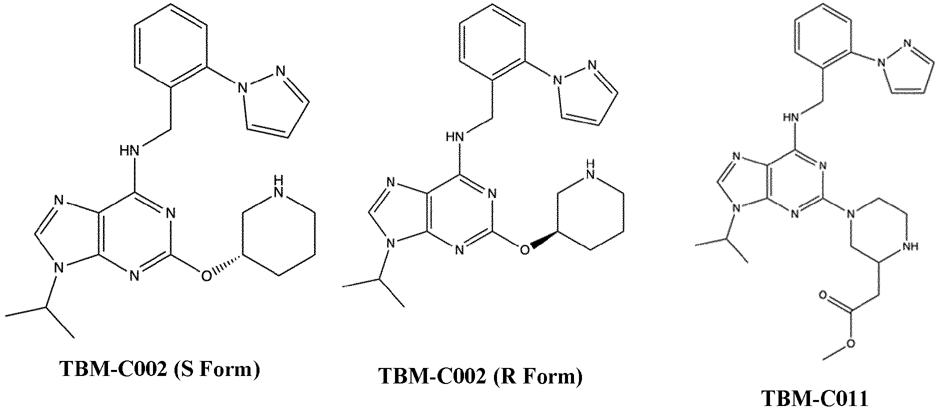

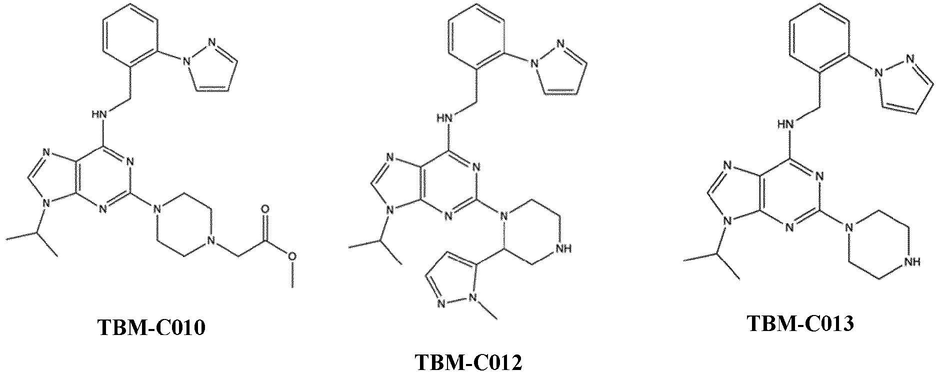

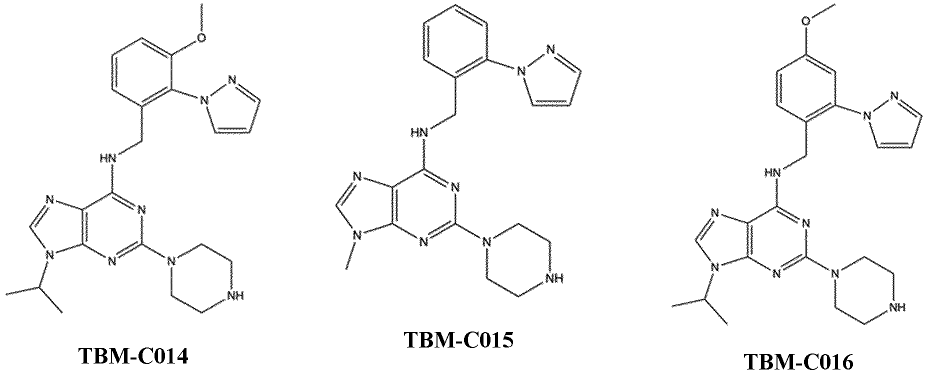

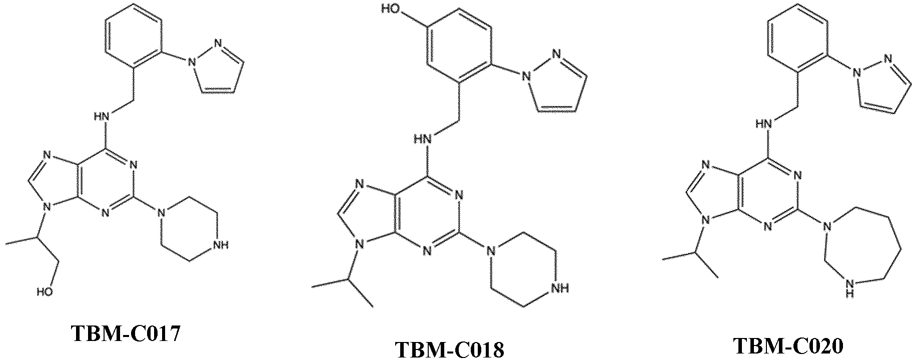

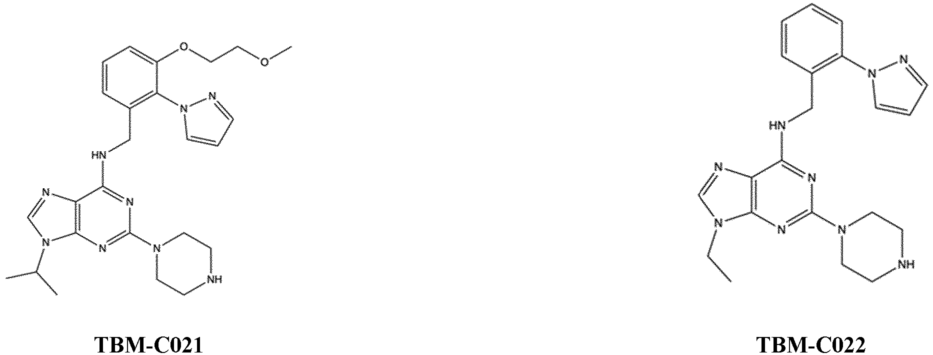

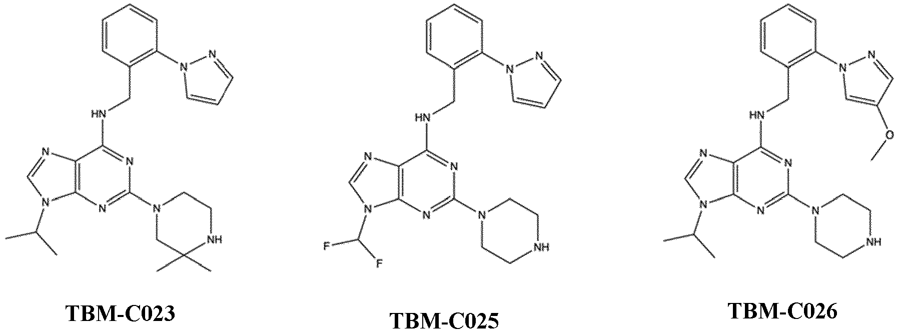

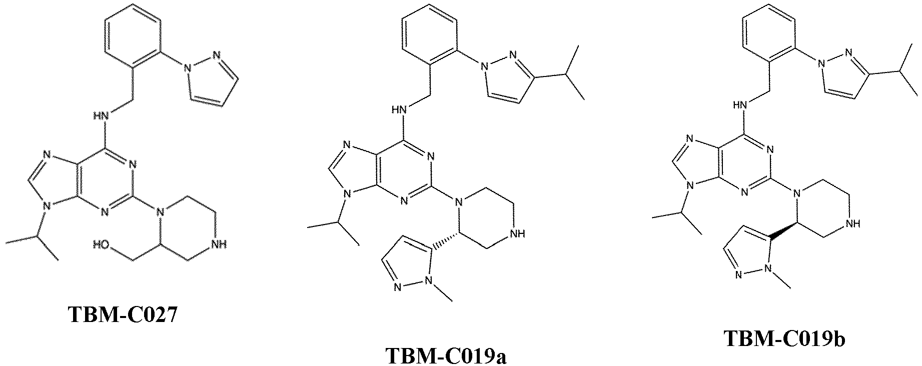

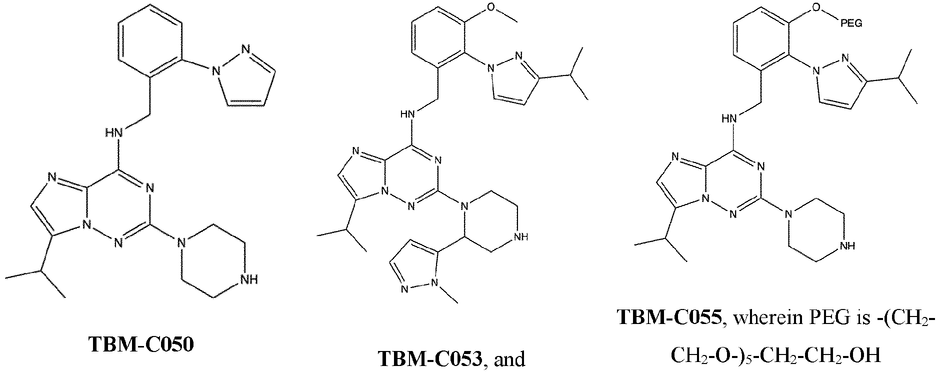

14. A medicament comprising the compound according to any of the preceding claims.

15. The compound according to any of claims 1 to 13 and/or the medicament according to claim 14 for use in the treatment of
(i) a proliferative disease, preferably cancer, more preferably pancreatic cancer, p53-based cancer, tumors with overexpressed Myc, tumors with upregulated cell cycle genes such as CyclinD, and/or cancer with high proliferation;
(ii) spreading of a virus; preferably Herpes viruses such as EBV and/or CMV;
(iii) inflammation; and/or
(iv) sepsis.
